(19) Europäisches Patentamt / European Patent Office / Office européen des brevets

(11) **EP 4 288 780 B1**

(12) **EUROPEAN PATENT SPECIFICATION**

(45) Date of publication and mention
of the grant of the patent:
**04.06.2025 Bulletin 2025/23**

(21) Application number: **22702054.2**

(22) Date of filing: **01.02.2022**

(51) International Patent Classification (IPC):
**G01N 33/574** (2006.01)   **B01L 3/00** (2006.01)
**G16B 40/00** (2019.01)

(52) Cooperative Patent Classification (CPC):
**G01N 33/57488;** G01N 2333/43534

(86) International application number:
**PCT/IB2022/050852**

(87) International publication number:
**WO 2022/167924 (11.08.2022 Gazette 2022/32)**

(54) **METHODS FOR CANCER DETECTION**

VERFAHREN ZUR KREBSERKENNUNG

MÉTHODES DE DÉTECTION DU CANCER

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **03.02.2021 IT 202100002294**

(43) Date of publication of application:
**13.12.2023 Bulletin 2023/50**

(60) Divisional application:
**24177632.7 / 4 404 201**

(73) Proprietors:
• **Disruptive Technological Advances In Life
Science S.r.l.- Società Benefit,
In forma abbreviata, D-TAILS S.r.l. SB
00161 Roma (IT)**
• **Fondazione Istituto Italiano di Tecnologia
16163 Genova (IT)**

(72) Inventors:
• **FOLLI, Viola
00046 Grottaferrata RM (IT)**
• **LANZA, Enrico
00184 Roma (IT)**

• **RICCO, Vincenzo
70125 Bari (IT)**

(74) Representative: **Predazzi, Valentina et al
Società Italiana Brevetti S.p.A.
Piazza di Pietra, 39
00186 Roma (IT)**

(56) References cited:
**EP-A1- 3 081 935**

• **DRAPER C. F. ET AL: "Menstrual cycle
rhythmicity: metabolic patterns in healthy
women", vol. 8, no. 1, 1 October 2018
(2018-10-01), XP055845831, Retrieved from the
Internet <URL:http://www.nature.com/articles/
s41598-018-32647-0> DOI: 10.1038/
s41598-018-32647-0**
• **LANZA ENRICO ET AL: "C. elegans-based
chemosensation strategy for the early detection
of cancer metabolites in urine samples", vol. 11,
no. 1, 24 August 2021 (2021-08-24),
XP055845840, Retrieved from the Internet
<URL:https://www.nature.com/articles/
s41598-021-96613-z> DOI: 10.1038/
s41598-021-96613-z**

**Description**

The present invention relates to methods for cancer detection using nematodes or nematodes GPCR receptors.

STATE OF THE ART

Olfaction is one of the primary mechanisms through which many animals adapt to the environmental changes. Olfactory receptors, which in all animals belong to the G-protein coupled receptors (GPCRs) family, play a crucial role in distinguishing the wide range of volatile or soluble molecules by directly binding them with high accuracy. Chemical sensitivity is particularly present in those organisms lacking long-range sensory mechanisms like hearing and vision. The genome of the nematode *Caenorhabditis elegans* possesses a remarkable number of genes encoding chemosensory receptors making it able to detect a similar number of odorants as mammals, despite the extremely low number of chemosensory neurons available.

The discovery of cheap and non-invasive diagnostic strategies for the early detection of cancer is an urgent priority.

Cancer is a disease that deeply alters the metabolome of the organism, potentially introducing its own characteristic waste products in biofluids. It has been reported that cancer tissues exude volatile compounds, and taking advantage of the extraordinary sense of smell of dogs and mice, previous studies allowed to demonstrate the existence of not yet known cancer-specific odorants in biological fluids. Animals perceive as odours a large variety of volatile molecules through olfactory perception. The ability to respond adaptively to any chemical alteration of the surrounding world is of pivotal importance for life and health in most animal species. Indeed, odours serve animals as environmental trackers to localize food and water sources, for nesting, and to discriminate conspecifics (pheromones) from individuals of other species (allelochemicals). Different compounds or different ratios of the same compound result in determining a specific odour fingerprint that may modulate such behaviours.

Odour sensing is mediated by a large family of proteins, known as G-protein coupled receptors (GPCRs). These are seventransmembrane domain proteins that upon binding their ligands, activate G-proteins, which in turn initiate intracellular signaling cascades. GPCRs are mainly expressed in the cilia of the olfactory neurons (ORNs). As yet, it is widely accepted, although with some exception, that in mammals each olfactory sensory neuron expresses uniquely one kind of olfactory receptor. The segregation of GPCRs on distinct olfactory cells ensures odour discrimination when the signals from functionally identical neurons are integrated into the olfactory bulb. On the contrary, in C. *elegans* each olfactory neuron presents multiple types of GPCRs on its membrane. Interestingly, despite the large number of genes encoding olfactory receptors (more than 1.000 including gustatory receptors), the nematode possesses a relatively small number of chemosensory neurons, 32, three of which are specialized to sense volatile molecules. Each olfactory neuron in C. *elegans* is therefore likely to detect a wider range of odorants with respect to mammalian ones. As a consequence, the combinatorial complexity of the odorant-receptor pair is extremely high in the nematode. Furthermore, the same GPCR can be expressed on distinct cells and the same odorant can bind different GPCRs on functionally different neurons and, depending on its concentration, it may elicit opposite behavioural outcomes. The behavioural response of C. elegans to odours is known as chemotaxis. A positive chemotaxis is observed when the worm is attracted by an odour and therefore moves towards it, a negative chemotaxis is observed when the worm avoids the odour and therefore moves away from it.

To date, the majority of the behavioural olfactory assays reported in C. *elegans* have been designed for single substances and responses have been assessed for a large variety of either volatile or soluble molecules including alcohols, ketones, aldehydes, esters, amines, sulfhydryls, organic acids, aromatic and heterocyclic compounds (Bargmann et al "Odorant-selective genes and neurons mediate olfaction in C. elegans" Cell, Vol. 74, August 13, 1993 pp 515-527). Many of the substances mediating attraction are natural metabolic products of bacteria, the food source of the nematode. However, the environmental stimuli C. *elegans* is exposed to are mostly complex mixtures of chemical compounds associated with competitive responses. Different ratios of attractive and repulsive components of a mixture can asymmetrically activate the neuronal architecture involved in the sensation of odours. As a result, C. *elegans* preference between two odours can be inverted in the presence of a third one (Iwanir, S. et al. Irrational behavior in c. elegans arises from asymmetric modulatory effects within single sensory neurons. Nat. communications 10, 3202 (2019); Cohen, D. et al. Bounded rationality in c. elegans is explained by circuit-specific normalization in chemosensory pathways. Nat. communications 10, 1-12 (2019)).

This highlights the importance of the chemical background on the C. elegans olfactory decision-making mechanism.

Recently, it was demonstrated that C. *elegans* displays attractive chemotaxis towards cancer urine samples while it is repelled by control samples (Hirotsu, T. et al. A highly accurate inclusive cancer screening test using Caenorhabditis elegans scent detection. PLoS One 10, e0118699 (2015); WO 2015/088039) in a highly accurate way. The ablation of olfactory neurons and the study on G protein $\alpha$ mutants suggest that the nematode responses are elicited by volatile compounds. Urine is notably a complex biofluid containing a large number of volatile compounds that differ in their chemical and physical features (i.e. molecular weight, polarity, hydrophobicity). The urine volatilome which includes the signatures of metabolic breakdown of food, contaminants, drugs, endogenous and bacterial by-products, is strictly related to the state of health of the individual. Altered concentrations in the volatilome have been found in spectra of GC/MS on cancer biofluids but, to date, the identification of specific cancer biomarkers through these methods may be problematic for two main reasons: the sensitivity up to the micromolar range and the wide battery of metabolites with altered concentrations which may not be cancer-specific. Among this wide number of molecules, the high sensitivity and specificity of the C. *elegans* olfactory system may guide the identification of the essential metabolic signature of cancer.

The fact that C. *elegans* performs positive chemotaxis towards cancer cell secretions, tissues and urine samples (dilution at 10-1) from people affected by cancer and negative chemotaxis towards samples from a control group was already reported by the work of Hirotsu (Hirotsu et al 2015 and WO 2015/088039), who also introduced the idea of exploiting this animal behaviour to discriminate between healthy people and people with cancer. The cited works also give evidence that olfactory neurons play a main role in driving the behavioural response. A further work (Ueda, Yuji, et al. "Application of C. elegans cancer screening test for the detection of pancreatic tumour in genetically engineered mice." Oncotarget 10.52 (2019): 5412.) applies the previous knowledge specifically targeting pancreatic cancer in mice.

In general, Hirotsu suggests that to detect cancer, it is possible to perform chemotaxis assays on a small population of C. *elegans* with urine samples. Positive chemotaxis indices are associated with cancer, while negative ones with a healthy condition. The reported sensitivity is 95.8% and the specificity is 95.0%. The positive group of urine samples (n=24 in total) was collected from people with different kinds of cancer (colon, rectal, gastric, pancreatic, oesophageal, breast and prostate) at different stages (I-IV according to the UICC criteria).

Both Hirotsu 2015 and WO 2015/088039, however, shows that the response of C. *elegans* to different biological cancer and non-cancer samples varies based on the sample and on the dilution of the same, demonstrating that there are several occasions in which the work is not capable to discriminate samples derived from cancer patients (or cancer cells) from samples derived from non-cancer patients (or cancer cells) as can be seen in figure S1 of Hirotsu 2015 as well as in figures 17 and 18 of WO 2015/088039. Additionally, although disclosing that in some cases olfactory neurons respond to cancer smell with a "high" response, the documents show that only the AWC neurons seem to provide a response that can possibly discriminate between cancer and non-cancer, but the decision whether the sample tested is or is not related to cancer is left to the interpretation of a "high" response of the neuron.

Therefore, although the use of C. *elegans* chemotactic or neuronal response to odorants appears to be an interesting basis for cancer detection, there are several pitfalls in the teachings of Hirotsu 2015 as well as in WO 2015/088039, that do not enable a reliable cancer detection for any cancer and for any sample or with any neuron as claimed by the authors and the documents provide no teachings on how to solve all the unanswered problems and on how to effectively use C. *elegans* for a reliable cancer detection.

Draper et al.: "Menstrual cycle rhythmicity: metabolic patterns in healthy women", SCIENTIFIC REPORTS, vol. 8, no. 1, 1 October 2018 discloses methods involving analysing serum hormones and urinary luteinizing hormone employing LC-MS and GC-MS for metabolomics and lipidomics.

SUMMARY OF THE INVENTION

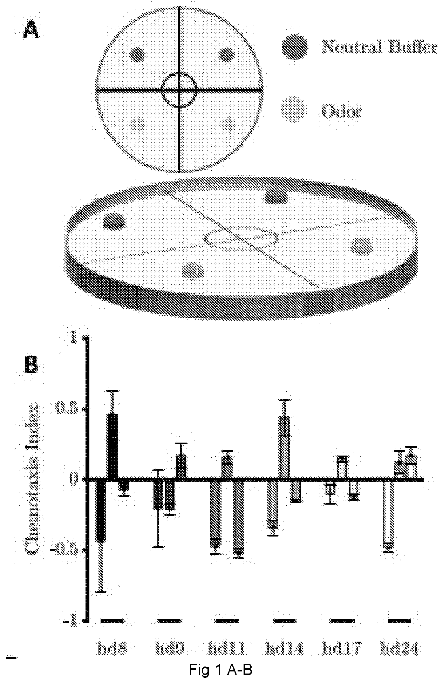

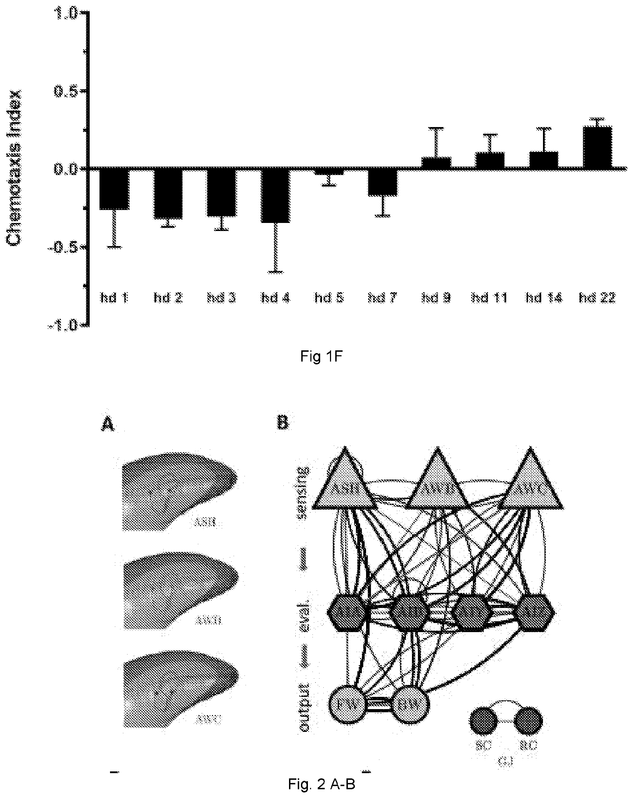

The authors of the present invention performed the same behavioural tests as Hirotsu et al 2015 on urine samples collected from healthy people and found conflicting results (see Figures 1 B and F): some control samples yield a negative chemotaxis index while others yield a positive one; multiple chemotaxis assays on the same samples may yield a positive index as well as a negative one. Figure 1 (B) shows that Chemotaxis assays on the same control samples may yield positive as well as negative results and Figure 1(F) shows that some control samples yield positive chemotaxis indices while others yield a negative one. However, according to what reported by Hirotsu, they should all yield a negative result. The protocol described by Hirotsu is thus incomplete and hardly reproducible.

When reporting the results about calcium imaging on the AWC neuron, the paper does not provide a statistically significant way to measure the neuronal response and to relate it to the discriminating preference displayed by C. *elegans* during chemotaxis assays in association with positive or control samples.

By performing calcium imaging, the work of Hirotsu indicates the neuronal response to urine collected from people with cancer could be used in order to detect cancer in urine. This kind of analysis involved a limited number of samples (2 positive samples from gastric cancer patients and 2 control ones) and did not include any sample collected from women with breast cancer. The samples of urine were administered at a dilution of 10-1 and for a time window of exposure to C. *elegans* of 10 seconds. In these conditions, the AWC neuron activates upon removal of positive samples and control samples as well, although the intensity of its activation is significantly lower in the latter case. The AWC neuron reportedly activates upon removal of attractants and may contribute to mediate attraction. Its activation in response to removal of control samples is lower in intensity, but not different in any other aspect from the activation in response to removal of positive samples, and as such, it cannot account for the preferential behaviour of C. *elegans* observed during chemotaxis.

Thus, Hirotsu's experimental conditions in calcium imaging tests on the AWC neuron cannot fully explain the discriminating ability of C. *elegans* displayed in chemotaxis assays on the two sets of samples. According to his results, the main contributor to the accurate chemotactic discrimination remains unidentified.

The authors of the present invention have surprisingly found that C. *elegans* attraction behaviour towards urine samples collected from women with cancer and of avoidance of those from healthy subjects is strongly influenced by the female hormone cycle and identified a window in which a chemotactic test on urine with C. *elegans* can reliably allow detection of urine samples deriving from women affected by cancer from samples derived from healthy controls and also introduced the requirement, in the test, that the subject to be tested must not take drugs altering the hormonal cycle.

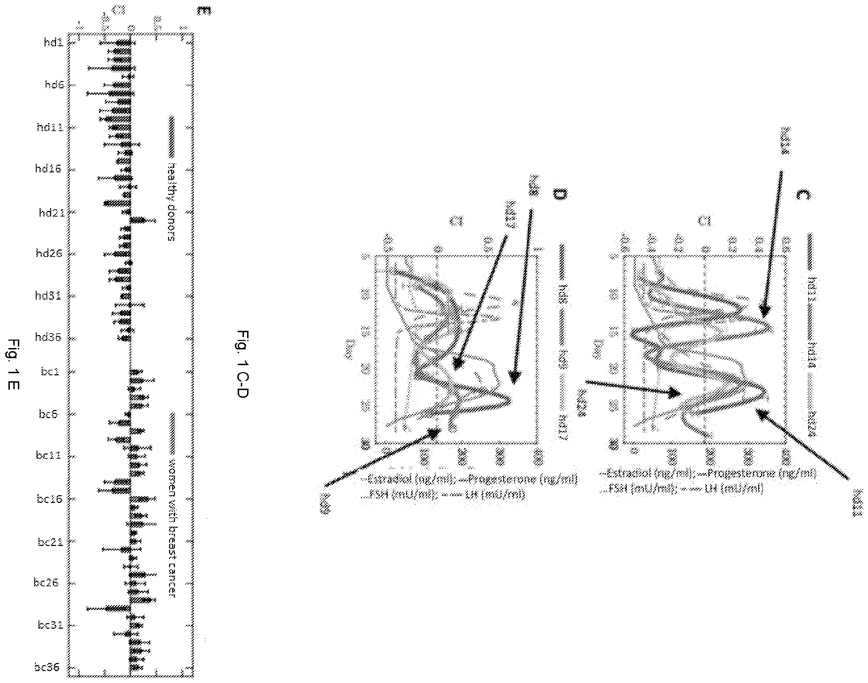

Figures 1 C and 1 D show chemotaxis indices obtained on urine samples collected over a month from six control subjects demonstrating that the chemotaxis indices are influenced by the hormonal cycle. In particular, during the estradiol and progesterone peaks indices become positive.

Additionally, the authors of the present invention identified receptors that are involved in cancer metabolites, indicating that a specific alteration of a restricted number of metabolites is sufficient for the highly accurate cancer discriminating behaviour of C. *elegans.* The authors of the present invention also identified a new measurement, herein defined as the "neuronal activation index" or NAI, which allows to quantify in a statistically significant manner the difference between the responses obtained for positive and negative samples in a calcium imaging test on C. *elegans* AWC neurons.

Object of the present inventions are:

- An *in vitro* method for detecting cancer in a woman, characterized by detecting cancer using, as an indicator, the reaction of free-living soil nematodes to the smell a urine sample of said woman; wherein the urine is collected at least two days after the last menstrual cycle and not later than five days after the same menstrual cycle;

- A microfluidic assay for detecting cancer in a subject, comprising the steps of: loading free-living soil nematodes in a microfluidic pulse arena chip that can be filled with chemicals in a timely controlled manner, said nematodes being transgenic nematodes in which the AWC$^{ON}$ neuron expresses a calcium indicator,

putting said nematodes in contact with a urine sample obtained by said subject;
assessing whether said contact activates or not the AWC$^{ON}$ neuron of said nematodes;
calculating the neuronal activation index NAI

$$NAI=2(AR\text{-}0.5)$$

where AR= *Nact*/*Ntot*
where $N_{act}$ is the number of nematodes responding with the activation of the AWC$^{ON}$ neuron and $N_{tot}$ is the number of viable nematodes tested for the same chemical stimulus;
wherein, when a NAI index >0 is obtained and/or when the AR value is >0.5 the subject is determined to have cancer and when a NAI index <0 is obtained and/or when the AR value is <0.5 the subject is determined to not have cancer.

Also disclosed is:

- A method for detecting cancer in which the activation of C. *elegans* AWA and AWC GPCR receptors activation is analysed upon contact with a biological fluid sample deriving from a subject, wherein the analysed receptors comprise *Sra-13; Str-2; Odr-10; Sra-17* and *Str-130* and when at least *Sra-13; Str-2; Odr-10; Sra-17* and *Str-130* are activated said subject is determined to have cancer;

- An apparatus for calculating a neuronal activation index NAI, comprising:

  - a housing for a microfluidic pulse arena chip, the chip being adapted to receive a number Ntot of free-living soil nematodes and a urine sample obtained by a subject;
  - a device for measuring the number Nact of nematodes responding with the activation of the AWCON neuron; and
  - a processing unit programmed for calculating said neuronal activation index NAI as NAI=2(AR-0.5),

  where AR= Nact/Ntot.

DETAILED DESCRIPTION OF THE DRAWINGS

Figure 1. *C.elegans* chemotaxis assays towards urine samples from healthy subjects and cancer patients(A) Population chemotaxis assay plate design. A 10 cm diameter Petri dish was used to conduct chemotaxis assays. The plate was divided into four quadrants, two odorant areas (+) and two control areas (-). One microliter of either cancer or control urine samples diluted at $10^{-1}$ was placed on the odorant areas (+). Nematodes were placed in the center of the plates and after 60 minutes a chemotaxis index (CI) was calculated as CI=(number of worms at the odorant areas (+)- number of worms at the control areas (-)) divided by the total number of worms. (B) The chemotaxis index of C. *elegans* is influenced by female hormones. Chemotaxis assays on urine collected from six healthy women (hd) during different days of the month, showed a variability in the chemotaxis index, suggesting an influence by female hormones. Error bars indicate SEM of three independent experiments. (C-D) C. *elegans* responses to urine collected from healthy donors is influenced by menstrual cycle rhythmicity. Chemotaxis assays were performed using urine collected from six healthy donors (hd), three times a week, starting two days after the end of the period until the next period. Data showed a positive correlation between CI and luteinizing hormone, follicular stimulating hormone and estradiol release preceding ovulation, and the progesterone peak during the luteal phase (Draper, C. et al. Menstrual cycle rhythmicity: metabolic patterns in healthy women. Sci. reports 8, 14568 (2018)). Moreover, a difference in the shape of the curves between the two groups of women was noticed. The pre-ovulation curve was narrower in group 1 (C) with respect to that of group 2 (D), which was larger. Error bars indicate SEM of three independent experiments. Gray curves indicate the fluctuations of hormone release during the menstrual cycle (reference graph modified from Draper, C. et al. Menstrual cycle rhythmicity: metabolic patterns in healthy women. Sci. reports 8, 14568 (2018).) (E) Bar plot of the resulting chemotaxis index (CI) values for each sample of the control (blue bars) and positive group (red bars) reported with the corresponding standard deviation;

Figure 1 (F) Some control samples yield positive chemotaxis indices while others yield a negative one.

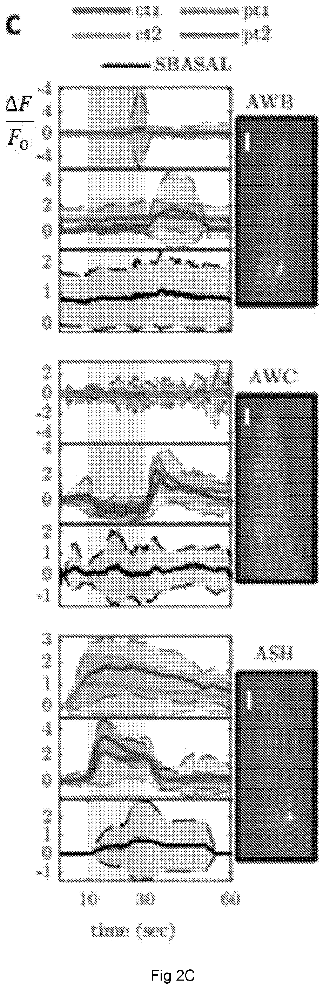

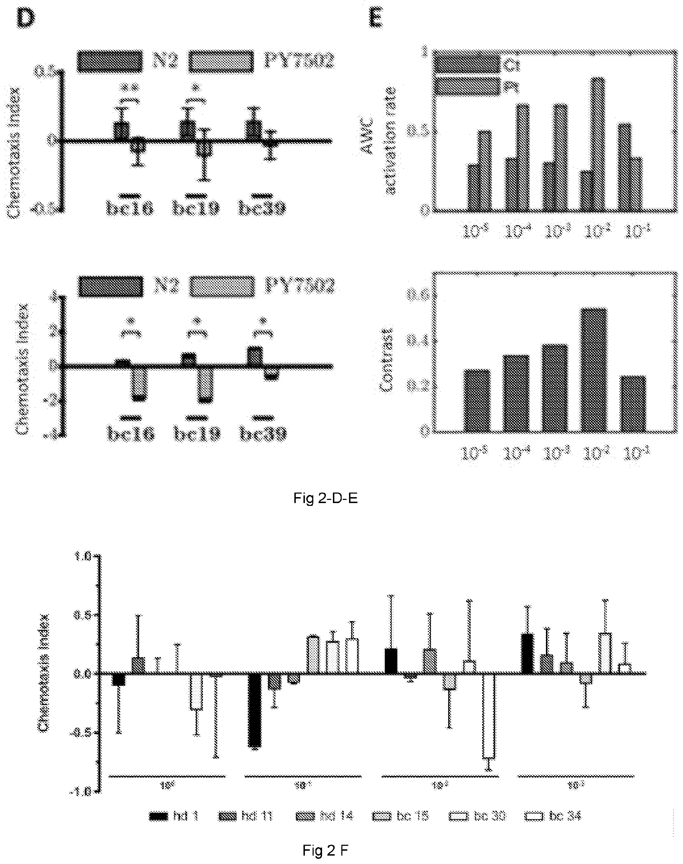

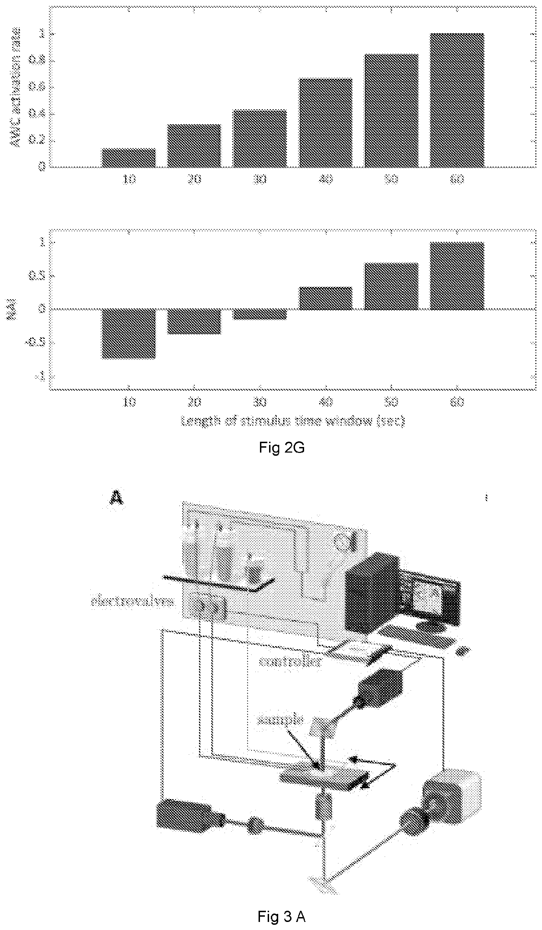

**Figure 2.** AWC is the main olfactory neuron mediating attraction towards cancer urine samples (A) Chemosensory neurons mediating aversive response (ASH and AWB) and attraction (AWC) located in the head. All neurons present processes extending to the tip of the nose. (B) Olfactory sensory neurons, downstream interneurons and motoneurons. (C) Mean calcium imaging traces normalized according to standard deviation (N=6) from (top to bottom) ASH, AWB and AWC neurons responding to control samples (repulsive), cancer urine samples (attractive) and to SBasal (neutral). Stimulation time is shaded in grey. ASH neurons respond to pressure changes related to flow switch, although showing a stimulus dependent activity. Traces from the AWB neurons do not seem to be reliable enough to explain the measured chemotaxis indexes. $AWC^{ON}$ neurons show reliable responses, making them the best candidate for comparisons with the CI. (D) AWC neurons sense cancer metabolites. Population (top) and single-animal (bottom) chemotaxis assays showed that AWC genetically ablated worms are unable to sense cancer metabolites. Bar plots indicate chemotaxis indices or chemotaxis scores in response to three informative cancer samples (bc16, bc19, and bc39). Error bars reported in population assays indicate SEM of three independent experiments (*p<0.05; **p<0.002, Student's t test). Error bars of single-animal chemotaxis assays indicate SEM of several tested animals (bc16: N2= 25, PY7502= 28; bc19: N2= 28, PY7502= 29; bc39: N2= 31, PY7502= 27) (*p<0.05, Mann-Withney test).(E) The top graph reports the activation rate of $AWC^{ON}$ neurons in response to removal of urine samples of cancer subjects (y-axis) or control samples for solutions with rising concentrations (from $10^{-5}$ to $10^{-2}$). The bottom graph shows the resulting contrast for each concentration. Concentration of $10^{-2}$ yields the highest activation rate (83.33%) and a reasonably good contrast between control and positive samples.

Figure 2F The 1:10 dilution was found to be the best concentration to discriminate between BC urine samples and controls. Several concentrations of urines were tested via population chemotaxis assays. The maximum attraction to BC samples (bc), as well as the greater avoidance towards controls (hd), peaked at a 1:10 dilution. Error bars indicate SEM of three independent experiments.

Figure 2G Preference inversion for control samples in AWC depends on the exposure time. AWC activation rates and corresponding NAIs obtained for the same control sample as a function of the length of the stimulus time (top graph and bottom graph respectively). The NAI highlights how the stimulus presented for 40 seconds or longer times elicits attractive-like responses in the majority of the tested nematodes, whereas shorter time-windows are associated with no response from most of the collected AWC traces.

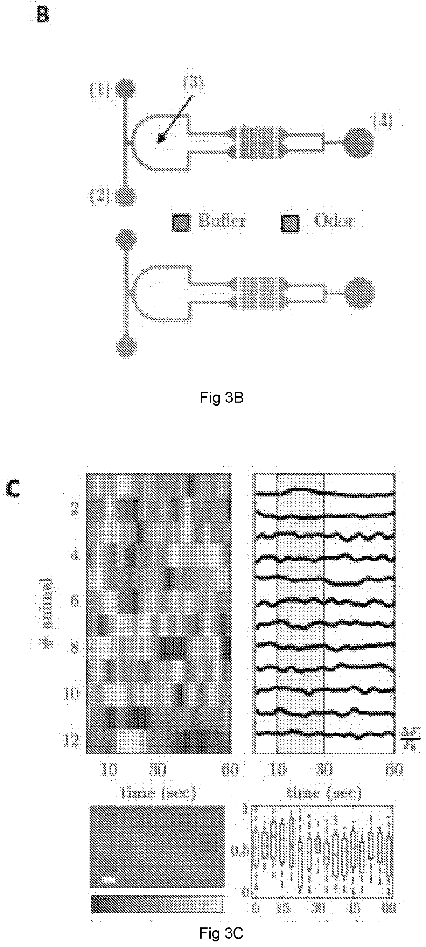

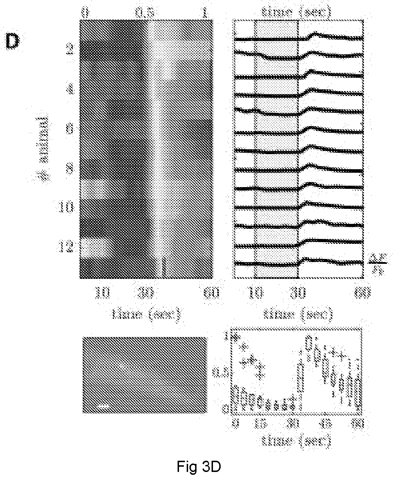

**Figure 3.** Wide-field imaging of neural activity upon chemical stimulation. (A) The sketch showing the experimental setup for simultaneous chemical stimulation via electro-valves and recording of neural responses via 470 nm blue excitation of GCaMP (blue line) and its emission (green line), and 660 nm light (red line) for transmission images. (B) Top view showing pulse arena geometry in two different conditions (buffer or odor filling).(C-D) Heatmaps and individual traces representing peak normalized neural responses (F/F0) across 12 animals and time from (C) healthy donors and (D) cancer subjects recorded on $AWC^{ON}$ neuron (pictures show a crop of a wide-field 4X fluorescence image on a head with a quiescent $AWC^{ON}$ neuron (top) and a chemically stimulated one (bottom)). Box plots show the corresponding population average peak F/F0. The central mark indicates the median, and the boxes indicate the 25th and 75th percentiles, respectively. The whiskers extend to the most extreme data points not considered outliers, and the outliers are plotted individually using the '+' symbol.

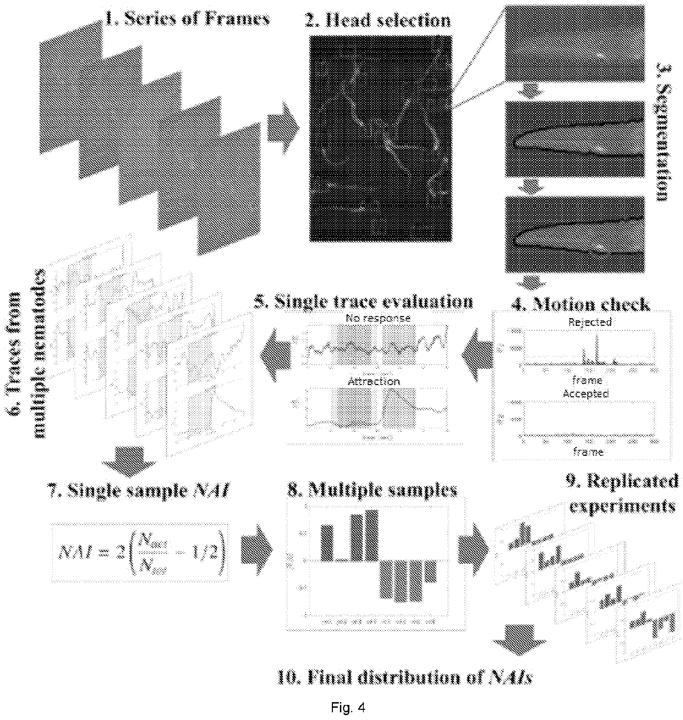

**Figure 4.** Pipeline of the post-processing processes. Starting from a series of frames (1), heads are selected and segmented for

$AWC^{ON}$ identification (2-3). If the nematode stays still during the acquisition, it will pass the motion check and the trace will be evaluated (4-6). The NAI is calculated considering all viable traces available in the acquisition (7). Each experimental run tests about 4 positive samples and 4 control ones (8). Averaging between different days of experiments (9) finally yields an average NAI and a standard deviation for each sample (10). More details are reported in the Methods section.

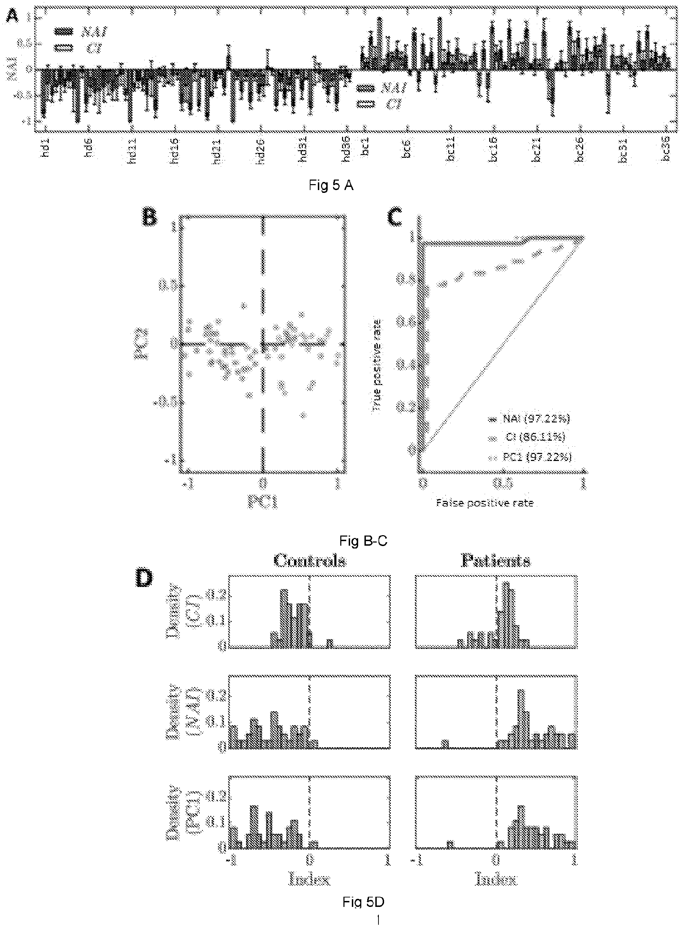

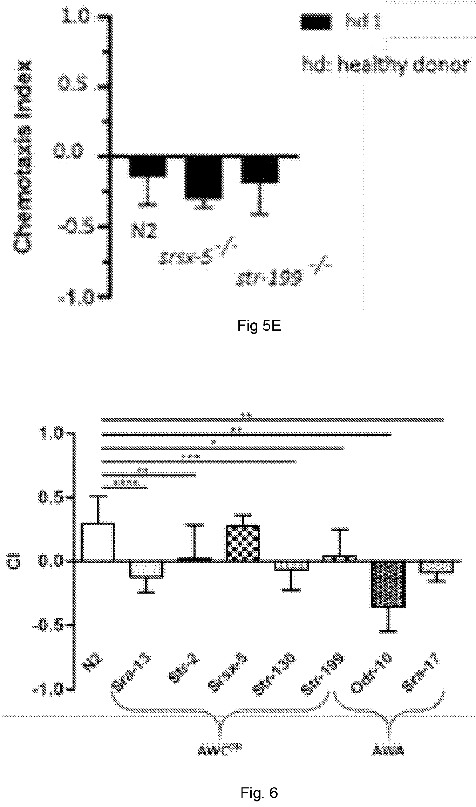

**Figure 5.** (A) Bar plot of the resulting NAI and CI values for each sample of the control (blue bars) and positive group (red bars) reported with the corresponding standard deviation calculated assuming a binomial distribution of the variables. Although both indexes show a clear tendency (positive values for cancer samples, negative values for control group), the accuracy and the contrast associated with the NAI is evidently higher if compared to the CI. (B) The scatter plot shows the first two principal components of the PCA analysis. The text boxes report the outcome of the analysis, which show that NAIs contribute to most of the separation between the points of the two groups. (C) ROC curves obtained for the CI (left graph), for NAI (center graph) and the first component of the principal component analysis (right graph). It is clear from the graphs that the calcium imaging measurements yield a better discrimination between the positive group and the control one. The first component of the PCA improves the discrimination power of the NAI, although it does not differ from it too much. (D) Distributions of the measured CI, NAI and PC1 (from top to bottom), for both the control group and patients (left and right graphs respectively for each row). Figure 5E Mutant animals carrying a deletion in the srsx-5 and str-199 genes behave as control worms. Animals lacking the SRSX-5 and STR-199 receptors behave as control animals (N2) also when tested with urine samples collected from healthy individuals (hd). Error bars represent SEM of three independent experiments.

**Figure 6.** Chemotaxis index of animals knock-out for the indicated gene Strains whose CI is significantly lower with respect to that of N2 are considered as strong candidates for sensing breast cancer metabolites. Statistical significance was calculated using unpaired Student's T-Test. Graph is presented as the average of assays performed using two different urine samples. Error bars represent SEM (* $P < 0.05$, ** $P < 0.01$).

## DETAILED DESCRIPTION OF THE INVENTION

The authors of the present invention have found that assays for detecting cancer, as the ones disclosed in Hirotsu, T. et al. PLoS One 10, e0118699 (2015); WO 2015/088039 were not repeatable, i.e. did not provide reliable and repeatable results in various cases, in particular in the identification of breast cancers.

The authors of the present invention have discovered that C. *elegans* reacts in different ways to samples collected from the same female individual depending on moment of the female hormonal cycle in which the samples were collected. The authors of the present invention have therefore identified a time window with respect to the female hormonal cycle for detecting cancer in a woman in assays wherein the reaction to the urine smell of C. *elegans,* or of a soil free-living nematode is used.

In the example section, the study carried out by the inventors is reported. The study, specifically targeted breast cancer at different stages (I-IV), and of both types (lobular and ductal) over 36 positive samples and 36 control samples (age and group matched). The same set of samples were tested in chemotaxis assays as well as in calcium imaging experiments.

In chemotaxis assays, the authors found a new protocol for urine collection and testing that allows to reproduce the results of Hirotsu, otherwise unreproducible. Urine samples collected over a month from 6 women were tested. The resulting chemotaxis indices suggested that *C. elegans's* chemotactic behaviour towards urine samples is influenced by the female hormonal cycle. In particular, peaks of progesterone and estradiol invariably produce an attractive response regardless of the health condition of the subject. The authors thus identified time windows in which chemotaxis assays yield reproducible results.

The present invention therefore provides
an *in vitro* method for detecting cancer in a woman, characterized by detecting cancer using, as an indicator, the reaction of free-living soil nematodes to the smell a urine sample of said woman; wherein the urine is collected at least two days after the last menstrual cycle and not later than five days after the same menstrual cycle.

It is known in the art that nematodes are an extremely broad phylum and that, although sharing a number of essential common features, roundworms can be parasitic or non-parasitic, the parasitic ones being adapted to insects, mammalians, fishes, plants and more, the non-parasitic (free living) ones being adapted to living in soil, water such as sea, lakes, rivers, ponds etc. As nematodes rely for their feeding and survival on smell, their sense of smell is highly developed and specialised. It is known, for example, that parasitic nematodes respond to odours in a very different manner compared to soil free-living ones. Therefore, all the embodiments of the invention (methods and assays) are is considered to be reasonably applicable to nematodes having life habits similar to *C. elegans,* such as free-living soil nematodes. In particular, the methods as well as the assays of the invention are considered to be further reasonably applicable to bacterial feeders free-living soil nematodes. In a preferred embodiment of the invention, the free-living soil nematode is C. *elegans.*

According to the description, the reaction to the smell of urine of the nematode can be a chemotactic reaction, a neuronal reaction or a receptor reaction of the nematode under observation. In an embodiment of the invention the reaction is a chemotactic reaction.

Preferably, for any embodiment of the present description in which the urine tested is collected from a woman, said woman to be tested should not, at the time of collection, take drugs altering the hormonal cycle or having recently taken drugs altering the hormonal cycle, as said drugs could alter the hormonal ratios and, consequently, the nematode's response.

According to the invention, when the response tested is the nematode's chemotactic response, the urine sample is preferably diluted 1:10.

The results reported in the example section below and in figure 2F, show, in fact, that $10^{-1}$ dilution of the urine used for the chemotactic assays provides the more accurate and reliable results.

According to the method of the invention in any of the embodiments described so far, when the free-living soil nematode as defined above shows a positive chemotactic response to the smell of said urine or, in other words, is attracted by said urine, the women is determined to have cancer.

According to another embodiment of the invention, in the in vitro method carried on soil free-living nematode as defined above, the reaction analysed is a neuronal reaction, in particular, an AWC$^{ON}$ neuron reaction.

According to the results provided in the example section, in a preferred embodiment the AWC$^{ON}$ neuron reaction is tested with urine sample in which the urine is diluted 1:100. Although the results summarised in figure 2E show that AWC$^{ON}$ neuron reaction analysis can provide reliable results at least of $10^{-2}$, $10^{-3}$, $10^{-4}$, $10^{-5}$, the best response is obtained at a urine dilution of $10^{-2}$.

The authors of the present invention have identified a new assay in which the neuron response of a nematode can be tested in an extremely reliable and precise manner.

The authors carried out calcium imaging experiments are in microfluidics, using a microfluidic chip to confine multiple nematodes in an arena that can be filled with chemicals in a timely controlled manner. The field of view of the camera used to record calcium traces included multiple heads and can thus record multiple traces simultaneously.

During these acquisitions, a neutral chemical (sBasal) filled the arena for the first 10 seconds and the last at least 30 seconds of the experiment, while the urine sample filled the arena between these two time windows. For each nematode, the mean fluorescent signal intensity detected for about 10 seconds in a time window starting 2 seconds after sample removal , $\langle I_{off} \rangle$, and the mean signal intensity detected for about 10 seconds in a time window ending 2 seconds before sample removal, $\langle I_{on} \rangle$, as well as the standard deviation in the same "on" time window, $\sigma_{on}$ were measured. The authors found that when the difference between $I_{off}$ and $I_{on}$ is three times higher than $\sigma_{on}$, namely $(I_{off} - I_{on}) > 3\,\sigma_{on}$, the response could be classified as an activation upon sample removal. In other terms, when $(I_{off} - I_{on}) > 3\,\sigma_{on}$, the chemical sensed by the nematode is perceived as an attractant.

To study the two groups of positive and control samples in this setting, the authors identified a new measurement, herein defined as the "neuronal activation index" or NAI, which allowed them to quantify in a statistically significant manner the difference between the responses obtained for positive and negative samples.

To build the *NAI* these steps were followed:

- Classify the response of a statistically significant number of nematodes according to the rule described in the previous paragraph $(I_{off} - I_{on} > 3\,\sigma_{on})$
- Count the number of responses classified as *attraction,* $N_+$, and the total number of the responses regardless of their classification, $N_{tot}$.
- Calculate the activation rate, AR, with the following formula $AR = N_+/N_{tot}$
- Calculate the *NAI* using the following formula: $NAI = 2\,AR - 1$

From its definition, it follows that the *NAI* ranges from -1 to +1, just like the chemotaxis index, *CI*. A positive *NAI* is associated with a majority of responses in which activation upon sample removal (attraction) is observed, while a negative *NAI* is to be associated with experiments in which the majority of nematodes did not respond, i.e. their responses measured through calcium imaging did not satisfy the following rule: $(I_{off} - I_{on} > 3\,\sigma_{on})$. A similar kind of quantification is obtained with the activation rate, but in this case, the AR ranges from 0 to 1, unlike the *CI* and the *NAI*. and values higher than 0.5 are to be associated with *attraction-associated* responses.

The *NAI* and the AR allowed the inventors to show how the best dilution value for the samples to be used in calcium imaging experiments is $10^{-2}$ (see Figure 1 C and D). This value is different from the one used in Hirotsu's calcium imaging experiments, in which the AWC showed a slight activation even after removal of control samples. This is in line with the fact that samples need to have a higher dilution value when administered via a liquid medium directly in contact with the assayed nematodes if compared to the dilution used in chemotaxis assays, in which odorants are detected by *C. elegans* either as airborne chemicals or through the supporting agar substrates.

The *NAI* and the AR allow us also to define the best stimulation time window for sample administration (see Figure 2G): from our experiments, we see that stimulation time windows that range longer than 40 seconds elicit an *attraction-like* response even in control samples. We thus suggest to use a time window of 20 seconds.

Finally, to predict the presence of cancer in a subject from urine samples, the authors showed that the behavioural response measured by the chemotaxis index, *CI*, with sample dilution at $10^{-1}$ is less accurate if compared to the evaluation based on the calcium imaging traces of AWC exposed for 20 seconds to a $10^{-2}$ dilution of urine samples and the *NAI* quantification (see Figure 5A).

The authors hence provide herein an effective stimulation protocol for microfluidic chip based calcium imaging experiments targeting the AWC neuron to detect cancer traces in urine samples exploiting *C. elegans* olfaction.

The main advantages of performing calcium imaging on AWC is a rise in the accuracy of the results and the lower amount of time involved in the test. To obtain these advantages, it is needed a strain of *C. elegans* expressing a calcium indicator specifically in the AWC neuron and to inspect it under an epifluorescence microscopy setup. Samples to be tested also need to be administered at a dilution value of $10^{-2}$ for a time window of 20 seconds and the results need to be quantified through the neuronal activation index, *NAI*. This is further ameliorated by the introduction of a new urine collection protocol to exclude false positives caused by the fact that the hormonal cycle interferes with the *C. elegans* cancer discriminating ability.

Therefore, a further object of the invention is a microfluidic assay for detecting cancer in a subject, comprising the steps of: loading free-living soil nematodes in a microfluidic pulse arena chip that can be filled with chemicals in a timely controlled manner, said nematodes being transgenic nematodes in which the AWC$^{ON}$ neuron expresses a calcium indicator,

putting said nematodes in contact with a urine sample obtained by said subject;
assessing whether said contact activates or not the AWC$^{ON}$ neuron of said nematodes; calculating the neuronal activation index NAI

$$NAI=2(AR\text{-}0.5)$$

where AR= *Nact*/*Ntot*
where $N_{act}$ is the number of nematodes responding with the activation of the AWC$^{ON}$ neuron and $N_{tot}$ is the number of viable nematodes tested for the same chemical stimulus;
wherein, when a NAI index >0 is obtained and/or when the AR value is >0.5 the subject is determined to have cancer and when a NAI index <0 is obtained and/or when the AR value is <0.5 the subject is determined to not have cancer.

In the assays of the invention suitable nematodes are nematodes of an appropriate dimension for a microfluidic system, such as *C. elegans* or other free-living soil nematodes, preferably feeding on bacteria, of dimensions similar to *C. elegans.*

Suitable microfluidic arenas are known to the skilled person. Behavioural arenas are described, by way of example, in Albrecht and Bargmann, Nature methods 2011, "High-content behavioral analysis of Caenorabditis elegans in precise spatiotemporal chemical environments". In particular the arena is described in the first paragraphs of "system design and overview" and depicted in figure 1 of the article. Details on the arena are also provided in Supplementary note 2 of the same article "microfluidic designs and optimization". The skilled person can easily prepare a microfluidic arena miniaturising the one described by Albrecth and Bargmann 2011. Other suitable microfluidics behavioural arenas on chips are described in the art, by way of example in Adriana San-Miguel and Hang Lu, Wormbook.org 2013 "Microfluidics as a tool for C. elegans research" , page 3 figure 2, as well as in EP3698133A1 disclosing a microfluidic chip (figure 5) described in detail in pages 4, 13 and 14.

Any microfluidic system suitable for simultaneously analysing the olfactory response to stimulus solutions of a number of C. elegans (also defined herein as behavioural arenas), having worm loading channel/s and channel/s for liquid inflow and outflow are suitable for the assay of the invention. The system according to the invention, allows the administration of solutions of interest (sampls, neutral solutions, other) to *C. elegans* in a timely precise manner. The behavioural arena is of a size, suitable to confining multiple nematodes under the field of view of a device for image acquisition, such as, e.g. a camera. The confinement can be easily obtained through barriers, whose distance from each other is sufficiently small to prevent nematodes from escaping, but also high enough to still allow solutions to flow through. The microenvironment in which the nematodes are confined requires a forest of pillars (preferentially, with constant spacing and consistent geometry). The pillars, which support the chip, need to be arranged with a spacing that grants an unperturbed swimming of *C. elegans.* Any epifluorescence microscope may be used to record neuronal activity a through fluorescence channel and/or the behavioural response through a transmission channel.

According to the assay of the invention,

said putting in contact with a urine sample obtained by said subject can be carried out by filling said arena with an olfactory neutral solution for a first period of time at the beginning and for a last period of time at the end of the assay and, and filling said arena with a urine sample of said subject for an intermediate period of time between said first and last period of time, the precedent filling being removed from the arena upon introduction of the subsequent one; and said assessing of whether said contact activates or not the AWC$^{ON}$ neuron of said nematodes is carried out by measuring the AWC$^{ON}$ neuron response during said last and during said intermediate periods of time and calculating the mean value $I_{on}$ of said measuring during said intermediate period of time, the mean value of said measuring during said last period of time $I_{on}$ and measuring the standard deviation $\sigma_{on}$ during said last period of time;
the AWC$^{ON}$ neurone being considered activated when,

$$I_{off} - I_{on} > 3\,\sigma_{on}$$

and
not activated when,

$$I_{off} - I_{on} < 3\,\sigma_{on}.$$

According to an embodiment of the invention, said first period of time is of 8-12 seconds (e.g. 10 seconds), said intermediate period of time is of 10-20 seconds and said last period of time is of at least 30 seconds.

The last period is applied in order to bring back the neuron to the baseline fluorescence. Any time longer than 30 is suitable but not necessary. The inventors have verified that 30 seconds are sufficient for reverting the neuron to the baseline fluorescence. No upper value for this time period is needed for the reasons above. In practical terms, in order to speed up the procedures, an upper value could be to a maximum of 60 seconds, therefore 30-60 seconds, however, in principle the upper value could be even of hours.

According to an embodiment of the invention, said $I_{on}$ is measured for a period of time of about 8-15 seconds, such as about 10 seconds, preferably at least after 2 seconds from the introduction of said urine sample.

According to a further embodiment, said $I_{off}$ is measured for a period of time of about 8-15 seconds, such as about 10 seconds, preferably at least after 2 seconds from the introduction of said olfactory neutral solution.

According to a further embodiment, said $I_{off}$ is measured for a period of time of about 10 seconds, at least after 2 seconds from the introduction of said olfactory neutral solution and said $I_{on}$ is measured for a period of time of about 10 seconds, at least after 2 seconds from the introduction of said urine sample.

As stated above, said urine sample can be, by way of example, at a $10^{-2}$, $10^{-3}$, $10^{-4}$ or $10^{-5}$ dilution. A preferred dilution is $10^{-2}$.

According to the findings disclosed above and in the example section below, also in the microarray assay of the invention, when the subject is a female, preferably said urine sample is collected at least two days after the last menstrual cycle and not later than five days after the same menstrual cycle.

As stated above, in the assay of the invention, the AWC$^{ON}$ neuron of the assayed nematodes expresses a calcium indicator. This can be done using transgenic worms coding for a gene expressing a calcium indicator on the neuron of interest.

Examples of the calcium indicator gene include: a Yellow Cameleon (YC) gene; a GCaMP gene such, as, by way of example, the gene expressing GCaMP3 (circularly permuted green fluorescent protein-calmodulin-M13 peptide version 3).

Methods for ligating the indicator to a receptor on the neuron of interest are well known in the art, such as methods of ligating an indicator gene immediately downstream of a promoter, microinjection methods and the like and are described, by way of example, in "Molecular Cloning: A Laboratory Manual (4th Edition)" (Cold Spring Harbor Laboratory Press (2012)), or other laboratory manuals. Alternatively, a DNA solution can be injected into the gonad of a nematode according to a known method (Mello, C. C., Kramer, J. M., Stinchcomb, D.& Ambros, V. Efficient gene transfer in C. elegans: extrachromosomal maintenance and integration of transforming sequences. EMBO J 10, 3959-3970 (1991).).

Additionally, the inventors have identified, as disclosed in the examples section and in figure 6, GPRC C. elegans receptors that are involved in the positive chemotactic response to cancer samples.

As reported in the experimental section, receptors AWA and AWC *Sra-13; Str-2; Odr-10; Sra-17* and *Str-130* receptors are involved in the positive chemotactic response to cancer samples observed in *C. elegans,* while *Srsx-5* and *Str-199* are not.

In fact, *C. elegans* mutants defective for the activity of each of *Sra-13; Str-2; Odr-10; Sra-17* and *Str-130* receptors have shown a lower chemotactic response (i.e. a lower chemotactic index) when compared to wild type *C. elegans* when stimulated with cancer patients urine, whereas no difference in chemotactic response between mutants and wild type was observed following the same stimulation on *C. elegans* mutants defective for the activity of each of *Srsx-5* and *Str-199.*

Therefore, object of the present disclosure (not claimed) is also a method for cancer detection in a subject comprising subjecting *C. elegans* AWA and AWC GPCR receptors to odorant stimuli with a biological fluid sample deriving from a subject, and analysing the stimulation of at least *Sra-13; Str-2; Odr-10; Sra-17* and *Str-130,* wherein, when at least *Sra-13; Str-2; Odr-10; Sra-17* and *Str-130* are activated said subject is determined to have cancer.

Additionally, the method can comprise analysis also of *Srsx-5* and/or *Str-199* receptors activity.

The disclosure (not claimed) hence also encompasses a method for cancer detection in a subject comprising subjecting *C. elegans* AWA and AWC GPCR receptors to odorant stimuli with a biological fluid sample deriving from a subject, and analysing the stimulation of at least *Sra-13; Str-2; Odr-10; Sra-17* and *Str-130,*

wherein the analysed receptors further comprise *Srsx*-5 or *Str-199* and when at least *Sra-13; Str-2; Odr-10; Sra-17* and *Str-130* are activated and *Srsx-5* or *Str-199* are not activated said subject is determined to have cancer; or wherein the analysed receptors further comprise *Srsx*-5 and *Str-199* and when at least *Sra-13; Str-2; Odr-10; Sra-17* and *Str-130* are activated and Srsx-5 and Str-199 are not activated said subject is determined to have cancer.

According to the disclosure, additional GPCR receptors that can be analysed are one or more of *Srsx-3; Srsx-37; Srt-7; Srt-28; Srt-29; Srt-45; Srt-47; Srx-1; Srx-47; Srab-7; Srab-9; Srab-13; Srab-16; Srv-34; Str-261, Srd-17; Sre-4, Sri-14; Srj-21; Srj-22.* In an embodiment said assessment of the GPCR receptors activation can be carried out using *Saccharomyces cerevisiae* strains comprising each, a DNA coding for one of said AWC and/or AWA GPCR receptors, coupled to *Saccharomyces cerevisiae* Gα subunit so to express a GPCR-Gα chimera, each of said yeast strains also comprising a pheromone-responsive fluorescent transcriptional reporter gene so that upon activation of the GPCR-Gα chimera, the pheromone-responsive fluorescent transcriptional reporter gene is activated and the fluorescent reporter protein is expressed in the yeast.

Suitable *Saccharomyces cerevisiae* strains can be prepared as described in WO2014/169336, or with any other recombinant DNA technology. By way of example, the protocol described by Kapolka et al in PNAS 2020, vol 117, no. 23 pp 13117-13126 "DCyFIR: a high-throughput CRISPR platform for multiplexed G protein-coupled receptor profiling and ligand discovery" can be adapted in order to express C. elegans GPCRs instead of human GPCRs. In this case, GPCRs can be CRISPR-integrated directly into the yeast *Saccharomyces cerevisiae* genome. In this system, the GPCR of the nematode can be coupled to the Gα subunit of the yeast using a Gα C-terminal chimera in which the last five residues of the Gα of the yeast are replaced with the last five residues of one of the four Gα of the nematode in the C-terminal (ODR3, GPA2, GPA3, GPA13). Each strain will also contain a pheromone-responsive fluorescent transcriptional reporter. The strains thus obtained can be tested with cancer patients urine samples. The activation of the GPCR-Ga pair due to the interaction with specific cancer metabolites will lead to the transcription of the fluorescent gene.

Therefore, according to the disclosure, said assessment is carried out by putting in contact with said sample distinct *Saccharomyces cerevisiae* strains comprising each, a DNA coding for one of said AWC and/or AWA GPCR receptors, coupled to *Saccharomyces cerevisiae* Gα subunit so to express a GPCR- Gα chimera, each of said yeast strains also comprising a pheromone-responsive fluorescent transcriptional reporter gene, said GPCR receptors being at least *Sra-13; Str-2; Odr-10; Sra-17* and *Str-130* , wherein, when emission of fluorescence is observed in at least in *Saccharomyces cerevisiae* strains expressing *Sra-13*-Gα; *Saccharomyces cerevisiae* strains expressing *Str-2*-Gα; *Saccharomyces cerevisiae* strains expressing *Odr-10*-Gα; *Saccharomyces cerevisiae* strains expressing *Sra-17*-Gα and *Saccharomyces cerevisiae* strains expressing *Str-130*-Gα *Sra-13; Str-2; Odr-10; Sra-17* and *Str-130* GPCR receptors are defined as activated.

According to the disclosure, additional *Saccharomyces cerevisiae* strains that can be used are one or more distinct *Saccharomyces cerevisiae* strains comprising each, a DNA coding for one of said AWC and/or AWA GPCR receptors, coupled to *Saccharomyces cerevisiae* Gα subunit so to express a GPCR- Gα chimera, each of said yeast strains also comprising a pheromone-responsive fluorescent transcriptional reporter gene, said AWC and/or AWA GPCR receptors being *Srsx-3; Srsx-37; Srt-7; Srt-28; Srt-29; Srt-45; Srt-47; Srx-1; Srx-47; Srab-7; Srab-9; Srab-13; Srab-16; Srv-34; Str-261, Srd-17; Sre-4, Sri-14; Srj-21; Srj-22.* Finally, the disclosure (not claimed) also provides an apparatus for calculating a neuronal activation index NAI, comprising:

- a housing for a microfluidic pulse arena chip, the chip being adapted to receive a number Ntot of free-living soil nematodes and a urine sample obtained by a subject;
- a device for measuring the number Nact of nematodes responding with the activation of the AWC$^{ON}$ neuron; and
- a processing unit programmed for calculating said neuronal activation index NAI as NAI=2(AR-0.5),

where AR= Nact/Ntot.

Devices for measuring the number of nematodes responding with the activation of a neuron are well known in the art. A suitable device is, e.g. described in detail in EP3698133A1 disclosing an apparatus for automatically detecting the neuronal response of C. elegans. The device is described in detail in figures 1 to 6 of the document. The device described in EP3698133A1 comprises a mechanical selection unit configured to select nematodes in adult stage from an initial nematode population obtaining an intermediate nematode population, a nematode optical selection unit configured to select from the intermediate population a final population of nematodes in adult stage and to select nematodes in young adult stage from nematodes in egg producing adult stage to be sent to a measurement unit configured to detect the response of nematodes of the final population to a stimulus of an odorant substance, the mechanical selection unit being connected to the optical selection unit by a connection channel with an at least three way branch and the optical selection unit being connected to the measurement unit by a loading microchannel. The selection units not being essential features of a suitable device although preferred. An example of a suitable device is disclosed in figure A and described in the experimental section "microfluidic device design for neuronal imaging".

The study reported below was approved by Santa Lucia Foundation Ethics Board. Written and informed consent was obtained from all participants before study enrolment.

EXAMPLES

**Sample collection and clinical characterization of breast cancer subjects**

We collected n=36 urine samples from women with breast cancer and n=36 urine samples from sex- and age-matched healthy donors. Both groups ranged between 25 and 88 years of age. Cancer features are reported in table 1. Briefly, 88.9% of cases are invasive ductal carcinoma, the most common form of breast cancer. To test the ability of *C. elegans* to early detect cancer, the majority of breast cancer patients (66.7%) were selected in the early stages of the disease.

**Chemotaxis towards women urine samples is affected by the hormone cycle**

To confirm that *C. elegans* displays avoidance towards urine samples from healthy donors, population chemotaxis assays were initially performed (Fig. 1, panel (A)) using samples from healthy women. A first set of assays carried out on samples from 10 independent women showed controversial results (Fig.1F). Surprisingly, opposite CI values were also observed in chemotaxis assays performed on independent samples collected from the same individual at different days along a period of one month (Fig. 1, panel (B)) wherein the collection days were randomly selected for each donor. These data suggested a major role of the female hormone cycle in modulating the chemotactic response of nematodes. To test this hypothesis, biological samples were collected three times a week from six healthy fertile, pill-free women, three of them being <30 years of age (group 1), the others being >40 years (group 2). Sample collection started two days following the end of the menstrual cycle and finished at the beginning of the next. A positive correlation between the chemotactic response of animals and both the follicular and periovulatory phases was observed (Fig. 1, panels (C)-(D)). While a peak in the estradiol release characterizes the former phase, the levels of the follicular stimulating (FSH) and luteinizing (LH) hormones promptly rise during the latter. Similarly, a positive CI was measured during the mid-luteal phase, which is characterized by a second wave of estrogen release associated with the progesterone peak. Interestingly, the shape of the pre-ovulation peaks was narrower within group 1 (younger women) compared to group 2 (eldest women), in agreement with the well-established changes in hormones' release with aging.

These findings demonstrate how the avoidance behaviour of *C. elegans* towards control urine samples is strongly influenced by the menstrual cycle. Such a hormone-dependent behaviour has not been previously reported, making data interpretation quite puzzling. In subsequent analyses, samples of fertile women have been collected during two relatively narrow and specific time-windows, *i.e.* a few days after the end of the period or between the follicular and luteal phases, in order to avoid false positive results.

**Table 1.** Breast cancer (BC) type/staging in the analyzed cohort.

| Tumour histology | number (%) |
|---|---|
| Age [median (range)] | 68.1 ±11.9 |
| Gender (male/female) | 0/36 |
| AJCC/UICC stage | |
| 0 | 2 (5.6%) |
| I | 22 (61.1%) |
| IIIa | 4 (11.1%) |
| IIIb | 1 (2.8%) |
| IIIc | 3 (8.3%) |
| IVa | 1 (2.8%) |
| undetermined | 3 (8.3%) |

(continued)

| TNM classification | |
| --- | --- |
| Primary tumor (T) | |
| Tx | 2 (5.6%) |
| T0 | 2 (5.6%) |
| Tis | 1 (2.8%) |
| T1 | 23 (63.9%) |
| T2 | 6 (16.7%) |
| T3 | 0 (0%) |
| T4 | 2 (5.6%) |
| Regional lymph nodes (N) | |
| Nx | 1 (2.8%) |
| N0 | 26 (72.2%) |
| N1 | 5 (13.9%) |
| N2 | 4 (11.1%) |
| Distant metastasis (M) | |
| M0 | 34 (94.4%) |
| M1 | 1 (2.8%) |
| Mx | 1 (2.8%) |
| Histologic type | |
| Invasive ductal carcinoma | 32 (88.9%) |
| Invasive lobular carcinoma | 4 (11.1%) |

**C. elegans is attracted by urine samples from women with breast cancer but avoids control samples**

Three cohorts were enrolled for this study: a first group included 36 women aged 38-92 years diagnosed with primary breast cancer (bc) as reported in table 1; a second group was composed by 36 apparently healthy and age-matched females (hd); a third small cohort involved 5 subjects with atypical ductal hyperplasia (at). Based on the aforementioned considerations, samples from fertile women were collected two days following the end of the menstrual phase.

In line with previous data indicating a dose-dependent effect of cancer-derived biological samples on *C. elegans* chemotaxis behaviour, we tested several concentrations of urine and found that the maximum attraction to breast cancer samples, as well as the greater avoidance towards controls, peaked at a dilution of $10^{-1}$ (Fig.2F). Population assays showed that *C. elegans* displays a significant preference for samples collected from women with breast cancer, whereas control urines behave as chemorepellents conferring an avoidance response (*P < 0.001; ANOVA, post-hoc Tukey's HSD) (Fig.1E and Fig.2F). The ability of the test to classify an individual as "affected" (*i.e.,* sensitivity) or "not affected" (*i.e.,* specificity) was 78% and 95%, respectively. The CI obtained for atypical ductal hyperplasia samples was also positive on average, even though the sample size of this group does not permit any statistical analysis. In two smokers, the analysis was also carried out after one smoke-free week, with no changes in the overall response, indicating that this is not affected by cigarette smoking. These data confirm and extend previous findings indicating that *C. elegans* hermaphrodites are able to detect breast cancer urines and discriminate them from control samples with a relatively high specificity and sensitivity.

**Analysis of the activity of AWC neurons greatly improves the accuracy in cancer screening**

Chemotaxis is the downstream outcome of the integration of several upstream signals from chemosensory neurons. The result of a chemotaxis assay may be altered by the presence of interfering stimuli of any nature that may originate from temperature, humidity and/or mechanical solicitations for instance. To effectively determine whether *C. elegans* perceives cancer metabolites and with which accuracy, calcium imaging proves to be a powerful tool because it allows to directly record the activity of upstream olfactory neurons activated by ligands regardless of the presence of concurrent cues sensed by other neurons. The dissection of the neural olfactory circuit in *C. elegans* through ablation, behavioral assays, calcium imaging, and the electron micrographs shows the existence of three main pairs of olfactory sensory neurons with winged cilia named AWA, AWB and AWC. AWA and AWC mediate attraction to volatile odorants while AWB mainly responds to repulsive compounds. The two AWC neurons are structurally similar but functionally different: AWC$^{ON}$ neuron expresses a chemoreceptor-encoding gene, *str-2,* missing in AWC$^{OFF}$, that instead expresses an alternative chemoreceptor gene, *srsx-3*. As a consequence, AWC[ON] neuron senses 2-butanone and acetone while AWC[OFF] neuron senses 2-3 pentanedione. This genetic and functional asymmetry is fundamental in *C. elegans* odour discrimination and it could play a pivotal role in cancer sensing. AWC olfactory neurons are activated by odor removal and inhibited in the persistent presence of attractants. After 30 minutes of exposure to odorants, adaptation occurs, abrogating the chemotactic response. Another neuron results to be a good candidate for mediating the avoidance behaviour towards urine samples collected from healthy subjects: the ASH, polymodal neurons, which are reported to be associated with aversive stimuli. All these neurons are located in the head of the nematode (see 3D reconstruction in figure2A). The olfactory neurons synapse onto several interneurons which act on motoneurons that initiate movements towards or away from the source of the stimulus (Fig. 2, panel B).

A major role of AWC[ON] and a minor one of AWA in mediating attraction towards diverse biological samples from different types of solid tumours has recently been shown. To better understand which neurons participate in the chemotaxis behaviour towards urine samples, the activity of AWA, AWB, AWC and ASH in response to a stimulation with cancer and control samples was recorded. To do this, transgenic worms expressing a calcium indicator on the neuron of interest, specifically GCaMP3 (circularly permuted green fluorescent protein-calmodulin-M13 peptide version 3) were used. These animals are challenged against a selected panel of well-known attractive odorants to demonstrate functional equivalence with the wild-type N2 strain (data not shown). The response of the aforementioned neurons to chemicals associated with either a strong negative chemotaxis index (for neurons sensing aversive stimuli) or a strong positive chemotaxis index (for neurons sensing attractants) was then tested. A urine sample that tested negative multiple times in chemotaxis assays was used as reference for strong repulsion while assessing the response of the AWB and ASH neurons. A urine sample that tested positive multiple times in the assays was used as reference for attraction for the AWC and the AWA neurons. Figure 2, panel C shows typical responses from three of the four candidate neurons. AWA neurons, being their role secondary, less reliable and redundant when compared to AWC neurons were excluded. Moreover, the AWA neuron expresses a lower number of chemoreceptors (9) if compared to those on AWC neurons (22) with two receptors in common with AWC: this makes it sensitive to a lower array of chemicals. Responses from the AWB neurons seem to not correlate with either addition or removal of attractant or repellent (although showing calcium related dynamics during the experiment) while the ASH neuron does not respond to either attractant or repellent removal/addition. The AWC[ON] neuron instead shows robust hyperpolarization during addition of cancer urine and strong depolarization upon its removal in a stereotyped fashion while it is often silent with healthy control samples. To further demonstrate the role of AWC in recognizing breast cancer metabolites, chemotaxis assays on the PY7502 strain, in which both the AWC neurons were genetically ablated were performed. Since the chemotactic response is known to be affected by the population density on an assay plate, odour preference of AWC-ablated animals are tested in both population and single-worm assays, by using three among the most informative cancer urine samples (*i.e.,* those associated with the higher CI). As expected and in line with previous findings, the data obtained show that AWC-ablated nematodes have a reduced ability to sense cancer metabolites (*$P < 0,05$, Mann-Withney test), indicating an essential role for the AWC amphid neurons in mediating this behaviour (Fig. 2, panel D). Together, these results suggest that the AWC[ON] neuron plays a determinant role in driving attraction towards cancer biofluids. However, because this neuron is associated with attraction, it cannot account for repulsion sensed by the nematodes. To measure the overall tendency of *C. elegans* to respond through the activation of the AWC[ON] neuron upon subtraction of a specific chemical, a new index, indicated as the neuronal activation index (NAI) was defined by the inventors:

$$NAI = 2(Nact/Ntot - 0.5) \quad (1)$$

where $N_{act}$ is the number of nematodes responding with the activation of the AWC[ON] neuron and $N_{tot}$ is the number of viable nematodes tested for the same chemical stimulus. This definition forces the index to range from -1 to 1 and allows us a direct comparison with the chemotaxis index (see Methods for details).

To define the optimal range of urine dilution at which the nematode sensitivity is maximized, various concentrations (from $10^{-1}$ to $10^{-5}$, pure sample is discarded because urine pH may interfere with the nematode preferences) of a subset of samples eliciting high both positive and negative index in chemotactic assays were tested. The value that maximizes the activation rate upon removal is $10^{-2}$ (83.33% of activation rate for positive samples). At this value, the contrast between the activation rates of positive and control samples is also the best one for the considered range (Fig. 2, panel E). To determine how *C. elegans* recognizes cancer urine samples from control ones, the neuronal activation index of AWC as a function of the length of the stimulus time for a control sample was investigated. The AWC activation rates and measure the corresponding NAIs for control samples as a function of the length of the stimulus time were recorded (Fig.2G). The NAI values highlight how the stimulus presented for longer times elicits attractive-like responses in the majority of the tested nematodes, whereas shorter time windows are associated with no response from most of the collected AWC traces. This suggests that metabolites eliciting the activation of AWC neurons are less concentrated in control samples rather than in samples from cancer patients. For high-throughput experiments in calcium imaging, the neuronal activity from multiple nematodes was simultaneously recorded. Nematodes were loaded onto a custom-designed microfluidic device with an all-liquid environment with a design based on the pulse arena of (Figs. 3(A-B)). The results were acquired for 36 women with breast cancer and 36 healthy subjects. Each sample has been tested in at least two different sessions, in which a minimum of 25 worms has been exposed to the odorant. This means that the NAI reported for every patient is obtained considering a minimum of 50 nematodes. Figure 3, panels (C-D) show typical traces obtained for one sample of both groups. To calculate the NAIs, a rule for the systematical identification of significant activation events in the AWC$^{ON}$ neuron was also defined. To do this, a custom script that extracts the fluorescence traces from the videos and evaluates them was employed. Figure 4 shows the pipeline of the post-processing process for an example image. More details are reported in the relative section of Methods.

Panel A of figure 5 shows a bar plot reporting for each sample the resulting NAI (left) and the CI (right). As expected, there is a clear tendency in the NAIs obtained through calcium imaging: cancer samples are associated with a positive NAI, while healthy subjects have a negative NAI, meaning that the first group elicits the activation of the AWC$^{ON}$ neuron as opposed to the second one, which does not trigger with the same frequency the chemical response in the neuron. Moreover, the measured NAIs are consistent with the results obtained in the chemotaxis assays, and with the assumption that the AWC$^{ON}$ neuron mediates chemical attraction, contributing to a positive CI. PCA analysis (panel B) considering both indexes shows a first component explaining the 90.97% of the total variance. Furthermore, the NAI contributes to more than the 97.31% of the first component, highlighting its significance as discriminator. When looking at the distributions of the CIs, the NAIs and PC1 (panel C, from top to bottom respectively), the use of a linear combination of the two indexes (as PC1) contributes to the removal of just a value centered in zero in NAI's index. This is probably due to the fact that the NAI lacks a corresponding N_ term, accounting for repelled nematodes in the CI. This makes it more sensitive to detect attraction, while it is unable to distinguish between a lacking response and repulsion. Linearly combining the NAI with a quantity that takes into account repulsion as well, allows to move the value centered in zero appearing in the NAI distribution to a value that is close to zero, but positive. The accuracy associated with calcium imaging experiments (97.18%) is significantly higher than the one obtained through chemotaxis (85.92%). The accuracy is defined as the proportion of true positives and true negatives among the total number of cases examined. The ROC curves as well, show how the NAI index is a better descriptor to discriminate between the two groups of samples (Panel D) than CI. Additionally, the NAI index is associated with a higher accuracy in discriminating urine samples of cancer group from the healthy one. The fact that the accuracy of the NAI evaluated only on the AWC$^{ON}$ neuron is higher than the one associated with the chemotaxis index supports the hypothesis that this neuron plays the main role in mediating attraction towards urine samples. For this to be true, urine samples eliciting attraction must contain chemicals at concentrations allowing them to interact with receptors on AWCs. This means that its receptors are good candidates as binding substrates for cancer related metabolites, providing chemo-physical constraints on the interacting metabolites and their relative abundances. On the other hand, the high accuracy associated with the AWC$^{ON}$ neuron also proves that chemical avoidance is not needed to discriminate between healthy samples and positive ones. However, *C. elegans* moves away from negative samples instead of just not showing attraction towards them. This suggests that there is a concurrent mechanism (yet not identified) contributing to the measured chemotaxis indexes, acting in a complementary way.

**Identification of the *C. elegans* GPCRs involved in the attraction towards cancer samples.**

It is well established that AWA and AWC neuron pairs mediate positive chemotactic responses to volatile odorants and as the predominant response to cancer urine is positive chemotaxis, we assumed that at least one cancer-metabolite receptor is expressed in the AWA and/or the AWC neurons. To test this hypothesis and identify the putative GPCRs responsible for sensing cancer urine metabolites, a small-scale pilot screen of AWC/AWA-GPCR mutants available from the *Caenorhabditis* Genetics Center, was performed via chemotaxis assays (Table 2). Mutant strains were exposed to two cancer urine samples. The ones that show a significantly reduced CI compared to that of wild-type animals to both samples, are to be considered putative cancer-sensing GPCRs. The mutant strains harbouring deletion of individual GPCRs expressed in AWC neurons was observed. Five mutant strains were tested. Three of them, *sra-13 (zh13), str-2 (ok3148)* and *str-130 (gk948599),* resulted in a significantly lower CI compared to N2, suggesting an involvement of these receptors in responding to breast cancer urine samples (Figure 6). Among these genes, *str-2* and *str-130* are expressed in AWC neurons exclusively, while *sra-13* is expressed in both AWA and AWC neurons (Table 2). Mutant strains, *srsx-5 (gk960578)* and *str-199 (gk949542),* resulted in a CI that is similar to that of wild-type and were therefore not contemplated as possible breast cancer-metabolite receptors (Figure 6). To rule out the possibility of the presence of an off-target mutation inserted during the original mutagenesis screen, these two mutant strains were also tested with control samples. The data obtained demonstrated that these KO worms behave as control animals, confirming that SRSX-5 and STR-199 are not involved in sensing BC odorants (Fig.5E). A couple of strains, *srt-26 (gk947940)* and *sri-14 (ok2865),* could not be tested because for the former, the deleted region encompasses a pseudogene, as described in WormBase release WS273, and the latter manifested locomotion defects in a thrashing assay (see details in section) impeding proper completion of the chemotaxis assay, which requires normal motility to be performed. It is worth to notice that this finding may highlight an unpredicted expression of the *sri-14* gene in neurons controlling locomotion, although an off-target effect occurred during the original mutagenesis screen cannot be excluded. On a second instance, despite the lower performance of AWA neurons in responding to urine samples, the results observed with *sra-13 (zh13)* mutant are suggestive of a putative contribution of such neurons and additional analysis on AWA-GPCR deletion mutants were therefore included. We examined a couple of commercially available strains, namely *sra-17ve511 [LoxP+myo-2::GFP+NeoR+LoxP)]* and *odr-10 (ky225)*. Interestingly, both strains showed a significant diminished CI towards cancer urine compared to N2 (Figure 6). This result suggests that despite the less robust calcium response of the AWA neurons, receptors on such neurons might have a role in recognising breast cancer metabolites, confirming the complexity underlying the olfactory system of C. *elegans.* Major information on the genes encoding the receptors herein analysed are summarized in Table 2.

**Table 2.** Summary of GPCRs functions and result in the chemotaxis assays. Information on expression profile and function of each receptor was acquired from WormBase version WS273. Comparison between N2 and mutant chemotaxis index is reported (p-value).

| Gene Name | Neuron | p-value |
| --- | --- | --- |
| *Sra-13* | AWA-AWC | 0.0015 (**) |
| *Str-2* | AWC$^{ON}$ | 0.02 (*) |
| *Odr-10* | AWA | 0.007 (**) |
| *Sra-17* | AWA | 0.001 (**) |
| *Str-130* | AWC | 0.014 (*) |
| *Str-199* | AWC | 0.09 |
| *Srsx-5* | AWC | 0.83 |
| *Sri-14* | AWC | Not tested (motility defects) |
| *Srt-26* | AWC | Not tested (pseudogene) |

**Discussion**

The ability of *C. elegans* to efficiently discriminate between subjects diagnosed with breast cancer and healthy controls by responding to urine, a bio-fluid that harbours an odour signature that is cancer-specific, is herein reported. The strongest evidence showing that *C. elegans* detects cancer smells in urine resides in the nematode binary behaviour observed in chemotaxis assays and calcium imaging analyses. Indeed, we measured with discrete accuracy (~97.18%) that urine from cancer subjects induced attraction while avoidance was observed towards control samples. The data in the present description show that the response of the nematode is dependent on the female hormone cycle, a phenomenon that has not been previously reported. According to these findings, two preferred and specific time-windows were herein identified for the collection of urine samples from fertile women, i.e. a few days after the end of the period or between the follicular and luteal phases, in order to avoid false positive results in the context of a diagnostic setting. Genetic analyses allowed to identify the sensory neurons involved in this behaviour as well as the individual GPCRs responsible for binding cancer metabolites. Chemotaxis assays with mutant strains lacking AWC neurons (AWC-killed strains), implied involvement of AWC olfactory neurons in attraction towards cancer urine samples. Even though mutation of *ceh-36* affects a second chemosensory neuron, ASE, its contribution in sensing cancer urine could not be confirmed as ASE is involved in the tasting response towards water-soluble metabolites. In order to measure a response towards this type of molecules, a different chemotaxis assay protocol is required. Experiments were performed over the period of one hour, which is not enough time for water-soluble molecules to diffuse on the plate and be detected by the worms. A one-hour long chemotaxis assay is sufficient for volatile components to diffuse and to be sensed. Under these circumstances the authors could not define a role for ASE neurons in sensing cancer urine. Nevertheless, the *ceh-36* mutants retained some capacity to discriminate between cancer urine and control samples suggesting that AWC is not the sole neuron detecting cancer smells. A possible contribution may derive from another olfactory neuron, AWA, also implicated in attraction towards volatile molecules. However, calcium imaging analyses showed a less robust and accurate activity of this neuron with respect to AWC in response to urine samples. In order to further dissect the molecular basis of this response, the role of G-protein coupled receptors which are responsible for odour sensing was investigated. C. *elegans* sensory neurons express a number of G-protein coupled receptors as opposite to mammalian neurons in which one neuron only expresses one type of receptors. AWC neurons express more than twenty different kinds of G-protein coupled receptors with unknown functions. Chemotaxis assays using AWC specific GPCR knock-out mutants showed involvement of three of these GPCRs, namely, *str-2, str-130* and *sra-13*, suggesting a role for these receptors in recognizing cancer related molecules.

AWA neuron expresses approximately half the amount of the olfactory receptors expressed in AWC. This may result in a lower number of binding candidate molecules and in a lower probability for the neuron to respond to urine. However, worms harbouring a deletion for *odr-10* and *sra-17*, both expressed in AWA, resulted to be defective in attraction towards cancer urine implicating that AWA olfactory neurons are also involved in cancer smells attraction. Of note, the molecular mechanisms underlying chemotaxis towards cancer metabolites is predicted to be more complex due to the possible synergistic effect exerted by multiple molecules. Furthermore, the analysis was restricted to GPCRs for which knock-out mutants were already available at the *Caenorhabditis* Genetics Center (CGC). To explore the contribution of additional receptors, future studies will be directed to generate novel null mutants of genes encoding GPCRs expressed in AWC and/or AWA sensory neurons via CRISPR-Cas9 genome editing. Control urine avoidance behaviour should also require G protein signaling pathways. However, ASH and AWB neurons, the main neurons mediating repulsion to volatile molecules, do not seem to induce avoidance from control urine. In fact, their neuronal dynamics are not reliable when the animals are exposed to control samples. The avoidance response from control samples could be the result of a complicated neuronal interaction involving several sensory neurons and a sophisticated signal transduction apparatus associated to possible co-expression and heterodimerization of GPCRs and could be more context-dependent with respect to the cancer attractive behaviour. The biochemical complexity in the chemoreceptors expression, may be an evolutionary adaptation mechanism to counterbalance the neuro-anatomical simplicity of *C. elegans* nervous system and its low plasticity. As a result, this complexity makes it more difficult to address the role of single olfactory neurons and to fully identify receptor complexes and their mechanisms in the overall functioning of the corresponding neuron. Nevertheless, the impressive accuracy (~97.18%) of AWC neurons suggests that the cancer urine blend comprises several components/metabolites present in distinctive relative abundances that robustly bind the olfactory receptors on these neurons. In conclusion, the results disclosed herein confirm tha t*C. elegans* is able to sense cancer-related metabolites in urine samples collected from women with breast cancer. This ability is strongly dependent on the hormonal cycle and we identify the time window of screening validity. Responses do not seem to be correlated with tumour stage: detection accuracy is high even at early stages. We demonstrate that calcium imaging on olfactory neurons yields more reliable results (accuracy of 97.18%) with respect to chemotaxis assays (85.9%), aside from being less time consuming. We verified through calcium imaging that the main contributor to the *C. elegans* cancer discriminating behaviour is the AWC$^{ON}$ chemosensory neuron and found a set of relevant GPCR receptors via genetic screens. The involvement of multiple GPCRs in the process suggests that multiple metabolites are sensed by *C. elegans.* Taken together, our results represent a proof of principle study for the exploitation of the incredible accuracy of the C. *elegans* chemosensory circuit to investigate the metabolic trace of cancer present in urine samples. Such a study may help designing a fast and cost effective diagnostic screening test for breast cancer, with a high reliability, based on the simple collection of biofluids.

Methods

### Study Design and Sample Collection

This study was approved by Santa Lucia Foundation Ethics Board. Written and informed consent was obtained from all participants before study enrolment. Midstream urine samples were collected from subjects with diagnosed primary breast cancer (n=36) before surgical procedure at the M.G.Vannini hospital in Rome. All cases were correlated with histology findings. Controls (n=36) were healthy, age-matched volunteers with no declared history of malignancy. Pregnant women were excluded from study enrolment, as were women taking the contraceptive pill or with uncontrolled bacteria, viral, or fungal infection. Upon arrival, all urine samples were centrifuged for 15 minutes at 4 °C, aliquoted in 0.2 ml tubes as single use samples and stored at -80 °C until measurements and analysis. On the day of the experiments, urine samples were diluted in either S-Basal for calcium imaging, or water for chemotaxis assays. A 1:100 dilution was used for calcium imaging measurements while 1:10 dilutions were utilized for odour screening chemotaxis assays. All dilutions were filtered before presenting them to the animals.

### Worm culture and strains

*Caenorhabditis elegans* strains were cultured at 20 °C on nematode growth medium (NGM) plates seeded with *Escherichia coli* OP50 as food source (Brenner, 1974). Wild-type animals used for this study were the Bristol N2 strain, obtained from the *Caenorhabditis Genetics Center* University of Minnesota, Minneapolis, MN, USA, along with a number of deletion mutant strains, including VC2123: *sri-14 (ok2865),* CX3410: *odr-10 (ky225),* AH159: *sra-13 (zh13),* RG3011: *sra-17 (ve511[LoxP+myo-2::GFP+NeoR+LoxP)] II,* VC20435: *srt-26 (gk947940),* VC30151: *srsx-5 (gk960578),* VC10129: *str-199 (gk949542),* VC40389: *str-130 (gk948599),* RB2316: *str-2 (ok3148).* The strain harbouring GCaMP in AWC$^{ON}$ neuron was the PS6374: AWC$^{ON}$-GCaMP syEx1240 *[str-2::GCaMP3+pha-1]; pha-1(e2123ts); him-5 (e1490),* a kind gift from Dr Alon Zaslaver, (The Hebrew University of Jerusalem, Jerusalem, Israel. The AWC-killed strain: PY7502, oyls85 *[ceh-36p::TU813*
*+ ceh-36p::TU814 + srtx-1p::GFP + unc-122p::DsRed])* was a kind gift from Dr Arantza Barrios (The University College London, London).

## *Behavioural assays*

For the chemotaxis assays, ten adult hermaphrodites were picked onto fresh NGM plates seeded with OP50 *E. coli* bacteria. The worms were left to lay eggs for five hours and then removed from the plates. Eggs were incubated at 20 °C and after four days adults were used for the test. Chemotaxis assays were performed as previously described (Bargmann et al., 1993) with minor modifications. Briefly, assay plates were prepared using 10 cm Petri dishes containing 11 ml of modified NGM (2% agar, 5mM KPO4 [pH6], 1mM CaCl2, 1mM MgSO4). Adult worms were washed twice in S-basal, once in distilled water and approximately 50-100 worms were placed in the centre of the plate right before starting the assay. The plates were divided into four quadrants referred to as the control and the odorant areas (Fig.1A). The control areas contained 1 $\mu$l of neutral buffer, whereas the odorant area contained 1 $\mu$l of either control or cancer urine sample, diluted in milliQ water at 1:10. One microliter of 1 M NaN3 was applied on each spot in order to immobilize the worms once they reached the area. After 60 minutes, animals were counted and a chemotaxis index (CI) was calculated as follows: (number of animals in the odorant area) - (number of animals in the control area)/Total number of animals tested. Chemotaxis assays were performed in triplicate on separate chemotaxis plates, to ensure assay reproducibility.

## *Single worm chemotaxis assay*

Single-animal chemotaxis assays were performed as previously described with some modifications. Assay plates were prepared using 5 cm Petri dishes containing 4 ml of modified NGM (2% agar, 5mM KPO4 [pH6], 1mM CaCl2, 1mM MgSO4). To visualize the trace of worms, the agar surface was dried for two hours just before placing the animals by opening the plate lid in the fume hood. Animals were placed on NGM plates without bacteria for one hour before the assay, then placed in the centre of the assay plate, 2 cm away from the source of urine samples. One agar plug was placed onto the lid of the plates and 1 $\mu$l of diluted sample was added to the plug immediately following the worm. After 20 minutes, the tracks left by the worms were visualized and the chemotaxis score was calculated as the sum of scores of the sectors through which the animal had travelled.

## *Thrashing Assay*

The locomotion behaviour was assessed through thrashing assays performed on wild type worms (N2, Bristol) and *sri-14 (ok2865)* mutant animals. Assays were carried out at 20°C on 35 mm OP50-free plates filled with 1 ml of M9 buffer (6 g/l Na 2 HPO$_4$, 3 g/l KH 2 PO$_4$, 5 g/l NaCl, 0.12 g/l MgSO$_4$). From plates containing synchronized young-adults, one animal at a time was transferred to a food-free NGM agar plate for 2 minutes to remove the bacteria and then assayed after 1 minute of acclimatization. Thrashes were counted for 20 seconds and multiplied by 3 to obtain an estimate per minute. A single thrash was defined as a complete change in direction of bending at the mid- body. At least 10 animals per genotype were assayed.

## Microfluidic device design for neuronal imaging.

For calcium imaging experiments, odour pulses were delivered by using microfluidic devices with a geometry based on the pulse arena Of with some variations introduced in the design: smoothing of the loading channel to facilitate nematode loading, removal of the free space between the layer of pillars and the inflow/outflow channels to avoid nematode piling up in the outflow side of the chip also for very slow flows, miniaturization of the overall dimension of the arena in order to allow recording of neuronal fluorescent signals from approximately 20 animals at once within a field of view of 3.25x3.25 mm$^2$ at 4x magnification of the CMOS camera. The chip, placed on a XY- microscope stage, is linked to two pressurized bottles through tygon tubes that drive the liquid directly in the inlet of the chip. The bottles are supplied by a gas line controlled with a gas adjustable regulator fixed at 100 mBar of pressure. The pressure value is validated to ensure a correct compromise between a fast odour switching in the arena (less than two seconds) and the avoidance of undesirable strong mechanical perturbations on the nematodes. Flow switch between odour and buffer is actuated slowly (about 2 sec) by motorized valves directly controlled by a software to prevent the inception of dangerous shock waves propagating in the tubes typically generated by a fast clogging of conventional solenoid valves. The electric command sent to the motorized valve is a PWM signal that allows to close them slowly without excessive stresses on the tube.

*Microfluidic device fabrication*

The microfluidic device is prepared by using the soft lithography process (Citazione witheside). The SU-8 (MicroChem 3000 series) structure is fabricated on a glass substrate by conventional photolithography to obtain a 50 microns thick monolayer microfluidic network. The high resolution photomask has been directly printed on the microfluidic network with a laser writer to ensure a resolution higher than 10000 dpi. Then, the 1:10 PDMS (Polydimethylsiloxane Silgard 184) mold replica is cured after the casting process and the holes for inlet/outlet are made with a 0.6 mm puncher to subsequently connect with external Tygon tubes. The PDMS layer is bonded on a microscope glass slide by using an air-plasma treatment (HARRIK PLASMA) and thermal recovery with hot plate. We first design the microfluidic network with CAD software and then produce the laser drawing of a high resolution glass photomask.

*Experimental setup for calcium imaging*

Calcium imaging recordings were made using a custom designed inverted microscope. A low magnification objective with a high-NA (4x/0.28 N.A.) and a sensitive low-noise CMOS camera with a large sensor area ($13 \times 13$ mm$^2$) allow to collect fluorescence signals from approximately 15-20 nematodes at once in a field of view of approximately $3.25 \times 3.25$ mm$^2$. Excitation light was reflected on the sample from a high power LED (470 nm - M470L2, Thorlabs, Newton, New Jersey) with a FITC excitation filter (MF475-35, CWL = 475 nm, BW = 35 nm, Thorlabs) and a condenser (ACL2520U-A, Thorlabs), using a dichroic mirror (MD498, Thorlabs). The signal was collected by a digital CMOS camera (ORCA-Flash4.0 C11440, Hamamatsu, Hamamatsu City, Japan) through the dichroic mirror with a pass-band FITC/TRIC filter (59004x, Chroma, Bellows Falls, Vermont) using a 20X objective with a numerical aperture of 0.28 (XLFLUOR4X/340, Olympus, Tokyo, Japan). Before starting the recordings, a transmission channel (illumination at 660 nm through a high power LED, M660L4, Thorlabs) allowed to select the visualized area of the arena through a motorized stage (MLS203-1, Thorlabs). To reduce phototoxicity and prevent photo-bleaching, the excitation LED and the camera shutter were synchronized so that the arena was illuminated only during the exposure time (100 ms). During the acquisition, buffer flows for the first 10 and last 30 seconds, while urine sample flows between these time windows for 20 seconds. The LEDs, the electrovalves and the camera were connected to a PC (Windows 10, 64-bit, Microsoft, Redmond, Washington) through a National Instruments controller (PCI-6221, National Instruments, Austin, Texas). A custom-made software in MATLAB (Mathworks, Natick, Massachusetts) was used for synchronized illumination, image acquisition, fluid delivery control and data recording.

*Ca$^{2+}$ imaging data analysis*

All steps of calcium imaging data analysis are made through custom MATLAB scripts. Acquired images are pre-treated by subtracting a mean background averaged over 20 background frames evenly recorded before and after the acquisition, and by applying an averaging filter for noise reduction. A custom-made GUI allows the user to select ROIs containing viable nematode heads in the frame acquired 2 seconds after removal of chemical stimulus, in which the probability of having visibly active neurons is higher. Nematodes that appear to have restrained access to the surrounding chemicals are discarded. In each ROI, the user is required to select the position of the AWC neuron. On the basis of this information, the script tracks neuronal positions throughout the video in both temporal directions by looking for the maximum intensity averaged over a $5 \times 5$ pixels area that may not be more than 30 pixels away from the neuronal position assigned in the previously processed frame. The resulting signal intensity at frame $i$, $I_i$, is calculated as $I_i = \Delta F_i / F_0$ where $\Delta F_i = F_i - F_0$. $F_i$ is the mean intensity of the segmented object representing the neuron. $F_0$ is the baseline value of fluorescence evaluated as the average of $F_i$ for $i = 1,2,..10$, a stimulus free time window, in which the neuron is quiescent. In case a neuron changes position in the ROI from a frame $i$ to its next one ($i+1$), the element-wise sum of the intensity difference, $ID_{i,i+1}$, between the ROIs in the two frames will vary much more if compared to a still video. Therefore, viable traces are identified as those ones in which the aforementioned difference satisfies the following condition: $ID_{i,i+1} < \overline{ID_i} + \sigma_{IDi}$, where $\overline{ID_i}$ and $\sigma_{IDi}$ are the mean value and the standard deviation respectively, calculated over all the *IDs* of the frames available in the range going from i-20 to i+20. This allows to exclude signals of moving neurons, whose evaluation may be affected by artefacts. All viable signals detected are visually validated by the user.

*Ca$^{2+}$ data quantification*

To quantify the activation rate of the AWC$^{ON}$ neurons upon removal of chemical stimulus from calcium imaging traces, we defined the neuronal activation index *(NAI)* as, $NAI = 2(Nact/Ntot -0.5)$, where $N_{act}$ is the number of nematodes responding with the activation of the AWC$^{ON}$ neuron and $N_{tot}$ is the number of viable nematodes tested for the same chemical stimulus. The subtraction of 1/2 forces symmetry around zero, while the prefactor 2 grants that $|NAI| \leq 1$, projecting the quantity into the range [-1,1]. From its definition, it follows that when $NAI > 0$, the majority of the tested nematodes experienced AWC$^{ON}$ activation, while a negative value is associated with the activation in a minority of them. For the systematic identification of significant activation events in the AWC$^{ON}$ neuron, a custom MATLAB script evaluates the calcium imaging signal intensity, $I$, for each neuron. If the difference between the mean signal intensity in a 10 second-long post-stimulus time-window, $I_{off}$, and the mean signal intensity in a 10 second-long time-window while on stimulus, $I_{on}$, is three times higher than the standard deviation of the signal in the time-window while on stimulus, $\sigma_{on}$, the response is associated with activation. It is associated
with a lack of response otherwise. All associations are then visually validated.

**Claims**

1. An *in vitro* method for detecting cancer in a woman, **characterized by** detecting cancer using, as an indicator, the reaction of free-living soil nematodes to the smell of a urine sample of said woman; wherein the urine is collected at least two days after the last menstrual cycle and not later than five days after the same menstrual cycle.

2. The *in vitro* method of claim 1 wherein said reaction is a chemotactic reaction.

3. The *in vitro* method of claim 2 wherein said urine sample is diluted 1:10.

4. The *in vitro* method of anyone of claims 1 to 3, wherein, when the free-living soil nematode shows a positive chemotactic response to the smell of said urine, the women is determined to have cancer.

5. The *in vitro* method of anyone of claims 1 to 4 wherein said reaction is an AWC$^{ON}$ neuron reaction.

6. The *in vitro* method of claim 5 wherein said urine sample is diluted 1:100 to 1:100000.

7. The *in vitro* method of anyone of claims 5 or 6, wherein, when said free-living soil nematode shows AWC$^{ON}$ neuron activation to the smell of said urine, the women is determined to have cancer.

8. A microfluidic assay for detecting cancer in a subject, comprising the steps of:

   loading free-living soil nematodes in a microfluidic pulse arena chip that can be filled with chemicals in a timely controlled manner, said nematodes being transgenic nematodes in which the AWC$^{ON}$ neuron expresses a calcium indicator,
   putting said nematodes in contact with a urine sample obtained by said subject, wherein said subject is a female and said urine sample is collected at least two days after the last menstrual cycle and not later than five days after the same menstrual cycle;
   assessing whether said contact activates or not the AWC$^{ON}$ neuron of said nematodes;
   calculating the neuronal activation index NAI

$$NAI = 2(AR\text{-}0.5)$$

   where AR= *Nact*/*Ntot*
   where $N_{act}$ is the number of nematodes responding with the activation of the AWC$^{ON}$ neuron and $N_{tot}$ is the number of viable nematodes tested for the same chemical stimulus;
   wherein, when a NAI index >0 is obtained and/or when the AR value is >0.5 the subject is determined to have cancer and when a NAI index <0 is obtained and/or when the AR value is <0.5 the subject is determined to not have cancer.

9. The microfluidic assay of claim 8 wherein

   said putting in contact with a urine sample obtained by said subject is carried out by filling said arena with an olfactory neutral solution for a first period of time at the beginning and for a last period of time at the end of the assay and, and filling said arena with a urine sample of said subject for an intermediate period of time between said first and last period of time, the precedent filling being removed from the arena upon introduction of the subsequent one; and
   said assessing of whether said contact activates or not the AWC$^{ON}$ neuron of said nematodes is carried out by measuring the AWC$^{ON}$ neuron response during said last and during said intermediate periods of time and calculating the mean value I$_{on}$ of said measuring during said intermediate period of time, the mean value of said measuring during said last period of time I$_{on}$ and measuring the standard deviation $\sigma_{on}$ during said last period of time;

the AWC$^{ON}$ neuron being considered activated when,

$$I_{off} - I_{on} > 3\ \sigma_{on}$$

and
not activated when,

$$I_{off} - I_{on} < 3\ \sigma_{on}.$$

10. The microfluidic assay of claim 9 wherein said first period of time is of 8-12 seconds, said intermediate period of time is of 10-20 seconds and said last period of time is of at least 30 seconds.

11. The microfluidic assay of claim 10 wherein said I$_{on}$ is measured for about 10 seconds and at least after 2 seconds from the introduction of said urine sample.

12. The microfluidic assay of claim 10 or 11 wherein said I$_{off}$ is measured for about 10 seconds and at least after 2 seconds from the introduction of olfactory neutral solution.

13. The microfluidic assay of anyone of claims 8 to 12 wherein said calcium indicator is the GCaMP3 (circularly permuted green fluorescent protein-calmodulin-M13 peptide version 3).

14. The microfluidic assay of anyone of claims 8 to 13 wherein said urine sample is at a $10^{-2}$, $10^{-3}$, $10^{-4}$ or $10^{-5}$ dilution.

15. The *in vitro* method of anyone of claims 1 to 7 or the microfluidic assay of anyone of claims 8 to 14, wherein the free-living soil nematode is *C. elegans*.

**Patentansprüche**

1. *In-vitro*-Verfahren zum Erkennen von Krebs bei einer Frau, **gekennzeichnet durch** das Erkennen von Krebs unter Verwendung, als einen Indikator, der Reaktion von frei lebenden Bodennematoden auf den Geruch einer Urinprobe der Frau; wobei der Urin mindestens zwei Tage nach dem letzten Menstruationszyklus und nicht später als fünf Tage nach dem gleichen Menstruationszyklus gesammelt wird.

2. *In-vitro*-Verfahren nach Anspruch 1, wobei die Reaktion eine chemotaktische Reaktion ist.

3. *In-vitro*-Verfahren nach Anspruch 2, wobei die Urinprobe auf 1 : 10 verdünnt wird.

4. *In-vitro*-Verfahren nach einem der Ansprüche 1 bis 3, wobei, wenn der frei lebende Bodennematode ein positives chemotaktisches Ansprechverhalten auf den Geruch des Urins zeigt, bestimmt wird, dass die Frau Krebs hat.

5. *In-vitro*-Verfahren nach einem der Ansprüche 1 bis 4, wobei die Reaktion eine AWC$^{ON}$-Neuronenreaktion ist.

6. *In-vitro*-Verfahren nach Anspruch 5, wobei die Urinprobe auf 1 : 100 bis 1 : 100000 verdünnt wird.

7. *In-vitro*-Verfahren nach einem der Ansprüche 5 oder 6, wobei, wenn der frei lebende Bodennematode eine AWC$^{ON}$-Neuronenaktivierung auf den Geruch des Urins zeigt, bestimmt wird, dass die Frau Krebs hat.

8. Mikrofluidischer Test zum Erkennen von Krebs bei einem Subjekt, umfassend die Schritte: Laden von frei lebenden Bodennematoden in einen mikrofluidischen Impulsarenachip, der in einer zeitlich gesteuerten Weise mit Chemikalien gefüllt werden kann, wobei die Nematoden transgene Nematoden sind, bei denen das AWC$^{ON}$-Neuron einen Calciumindikator exprimiert, Inkontaktbringen der Nematoden mit einer Urinprobe, die von dem Subjekt erhalten wird, wobei das Subjekt weiblich ist und die Urinprobe mindestens zwei Tage nach dem letzten Menstruationszyklus und nicht später als fünf Tage nach dem gleichen Menstruationszyklus gesammelt wird;

Ermitteln, ob der Kontakt das AWC$^{ON}$-Neuron der Nematoden aktiviert oder nicht;
Berechnen des neuronalen Aktivierungsindex NAI

$$NAI = 2(AR-0{,}5)$$

wo AR = Nact/Ntot
wo $N_{act}$ die Anzahl von Nematoden ist, die auf die Aktivierung des AWC$^{ON}$-Neurons ansprechen, und $N_{tot}$ die Anzahl von lebensfähigen Nematoden ist, die auf den gleichen chemischen Reiz geprüft wurden;
wobei, wenn ein NAI-Index von >0 erhalten wird und/oder wenn der AR-Wert >0,5 ist, bestimmt wird, dass das Subjekt Krebs hat, und wenn ein NAI-Index von <0 erhalten wird und/oder wenn der AR-Wert <0,5 ist, bestimmt wird, dass das Subjekt keinen Krebs hat.

9. Mikrofluidischer Test nach Anspruch 8, wobei

das Inkontaktbringen mit einer Urinprobe, die von dem Subjekt erhalten wird, durch ein Füllen der Arena mit einer olfaktorisch neutralen Lösung für einen ersten Zeitraum an dem Anfang und für einen letzten Zeitraum an dem Ende des Tests und das Füllen der Arena mit einer Urinprobe des Subjekts für einen dazwischen liegenden Zeitraum zwischen dem ersten und dem letzten Zeitraum durchgeführt wird, wobei die vorherige Füllung bei einer Einführung der nächsten einen aus der Arena entfernt wird; und
das Ermitteln, ob der Kontakt das AWC$^{ON}$-Neuron der Nematoden aktiviert oder nicht, durch ein Messen des AWC$^{ON}$-Neuronenansprechverhaltens während des letzten und des dazwischen liegenden Zeitraums und ein Berechnen des Mittelwerts $I_{on}$ des Messens während des dazwischen liegenden Zeitraums, des Mittelwerts des Messens während des letzten Zeitraums $I_{on}$ und das Messen der Standardabweichung $\sigma_{on}$ während des letzten Zeitraums durchgeführt wird;
das AWC$^{ON}$-Neuron als aktiviert betrachtet wird, wenn

$$I_{off} - I_{on} > 3\,\sigma_{on}$$

und
als nicht aktiviert, wenn

$$I_{off} - I_{on} < 3\,\sigma_{on}.$$

10. Mikrofluidischer Test nach Anspruch 9, wobei
der erste Zeitraum 8-12 Sekunden, der dazwischen liegende Zeitraum 10-20 Sekunden und der letzte Zeitraum mindestens 30 Sekunden beträgt.

11. Mikrofluidischer Test nach Anspruch 10, wobei $I_{on}$ für etwa 10 Sekunden und mindestens nach 2 Sekunden ab der Einführung der Urinprobe gemessen wird.

12. Mikrofluidischer Test nach Anspruch 10 oder 11, wobei $I_{off}$ für etwa 10 Sekunden und mindestens nach 2 Sekunden ab der Einführung der olfaktorisch neutralen Lösung gemessen wird.

13. Mikrofluidischer Test nach einem der Ansprüche 8 bis 12, wobei der Calciumindikator das GCaMP3 (zirkulär permutiertes grün fluoreszierendes Protein-Calmodulin-M13-Peptid Version 3) ist.

14. Mikrofluidischer Test nach einem der Ansprüche 8 bis 13, wobei die Urinprobe in einer Verdünnung von $10^{-2}$, $10^{-3}$, $10^{-4}$ oder $10^{-5}$ vorliegt.

15. *In-vitro*-Verfahren nach einem der Ansprüche 1 bis 7 oder mikrofluidischer Test nach einem der Ansprüche 8 bis 14, wobei der frei lebende Bodennematode *C. elegans* ist.

**Revendications**

1. Procédé *in vitro* permettant de détecter un cancer chez une femme, **caractérisé par** la détection de cancer à l'aide, en guise d'indicateur, de la réaction de nématodes du sol vivant librement à l'odeur d'un échantillon d'urine de ladite femme ; dans lequel l'urine est collectée au moins deux jours après le dernier cycle menstruel et pas plus tard que cinq jours après ce même cycle menstruel.

2. Procédé *in vitro* selon la revendication 1 dans lequel ladite réaction est une réaction chimiotactique.

3. Procédé *in vitro* selon la revendication 2 dans lequel ledit échantillon d'urine est dilué 1:10.

4. Procédé *in vitro* selon l'une quelconque des revendications 1 à 3, dans lequel, lorsque le nématode du sol vivant librement montre une réponse chimiotactique positive à l'odeur de ladite urine, il est déterminé que la femme souffre d'un cancer.

5. Procédé *in vitro* selon l'une quelconque des revendications 1 à 4 dans lequel ladite réaction est une réaction de neurone AWC$^{ON}$.

6. Procédé *in vitro* selon la revendication 5 dans lequel ledit échantillon d'urine est dilué 1:100 à 1:100 000.

7. Procédé *in vitro* selon l'une quelconque des revendications 5 ou 6, dans lequel, lorsque ledit nématode du sol vivant librement montre une activation de neurone AWC$^{ON}$ à l'odeur de ladite urine, il est déterminé que la femme souffre d'un cancer.

8. Dosage microfluidique permettant de détecter un cancer chez un sujet, comprenant les étapes consistant à : charger des nématodes du sol vivant librement dans une puce arène à impulsions microfluidiques qui peut être remplie avec des substances chimiques d'une manière maîtrisée dans le temps, lesdits nématodes étant des nématodes transgéniques dans lesquels le neurone AWC$^{ON}$ exprime un indicateur de calcium, placer lesdits nématodes en contact avec un échantillon d'urine obtenu par ledit sujet, dans lequel ledit sujet est une femme et ledit échantillon d'urine est collecté au moins deux jours après le dernier cycle menstruel et pas plus tard que cinq jours après ce même cycle menstruel ;

   le fait d'évaluer si ledit contact active ou non le neurone AWC$^{ON}$ desdits nématodes ;
   le calcul de l'indice d'activation neuronale NAI

$$NAI=2(AR-0,5)$$

   où AR= Nact/Ntot
   où $N_{act}$ est le nombre de nématodes répondant avec l'activation du neurone AWC$^{ON}$ et $N_{tot}$ est le nombre de nématodes viables testés pour le même stimulus chimique ;
   dans lequel, lorsqu'un indice NAI >0 est obtenu et/ou lorsque la valeur AR est >0,5 il est déterminé que le sujet souffre d'un cancer et lorsqu'un indice NAI <0 est obtenu et/ou lorsque la valeur AR est <0,5 il est déterminé que le sujet ne souffre pas d'un cancer.

9. Dosage microfluidique selon la revendication 8 dans lequel

   ladite mise en contact avec un échantillon d'urine obtenu par ledit sujet est effectuée en remplissant ladite arène avec une solution neutre olfactivement pendant un premier laps de temps au début et pendant un dernier laps de temps à la fin du dosage et, en remplissant ladite arène avec un échantillon d'urine dudit sujet pendant un laps de temps intermédiaire entre lesdits premier et dernier laps de temps, le remplissage précédent étant retiré de l'arène lors de l'introduction du suivant ; et
   ledit fait d'évaluer si ledit contact active ou non le neurone AWC$^{ON}$ desdits nématodes est effectué en mesurant la réponse du neurone AWC$^{ON}$ pendant ledit dernier laps de temps et pendant ledit laps de temps intermédiaire et le calcul de la valeur moyenne $I_{on}$ de ladite mesure pendant ledit laps de temps intermédiaire, de la valeur moyenne de ladite mesure pendant ledit dernier laps de temps $I_{on}$ et la mesure de l'écart type $\sigma_{on}$ pendant ledit dernier laps de temps ;
   le neurone AWC$^{ON}$ étant considéré comme activé lorsque,

$$I_{off} - I_{on} > 3\,\sigma_{on}$$

et
non activé lorsque,

$$I_{off} - I_{on} < 3\,\sigma_{on}.$$

**10.** Dosage microfluidique selon la revendication 9 dans lequel ledit premier laps de temps va de 8 à 12 secondes, ledit laps de temps intermédiaire va de 10 à 20 secondes et ledit dernier laps de temps est d'au moins 30 secondes.

**11.** Dosage microfluidique selon la revendication 10 dans lequel ledit $I_{on}$ est mesuré pendant environ 10 secondes et au moins après 2 secondes suivant l'introduction dudit échantillon d'urine.

**12.** Dosage microfluidique selon la revendication 10 ou 11 dans lequel ledit $I_{off}$ est mesuré pendant environ 10 secondes et au moins après 2 secondes suivant l'introduction de la solution neutre olfactivement.

**13.** Dosage microfluidique selon l'une quelconque des revendications 8 à 12 dans lequel ledit indicateur de calcium est le GCaMP3 (peptide circulairement permuté de protéine fluorescente verte-calmoduline-M13 version 3).

**14.** Dosage microfluidique selon l'une quelconque des revendications 8 à 13 dans lequel ledit échantillon d'urine est à une dilution de $10^{-2}$, $10^{-3}$, $10^{-4}$ ou $10^{-5}$.

**15.** Procédé *in vitro* selon l'une quelconque des revendications 1 à 7 ou dosage microfluidique selon l'une quelconque des revendications 8 à 14, dans lequel le nématode du sol vivant librement est C. *elegans*.

Fig 1 A-B

Fig. 1 C-D

Fig. 1 E

Fig 1F

Fig. 2 A-B

Fig 2C

Fig 2-D-E

Fig 2 F

Fig 2G

Fig 3 A

B

Fig 3B

C

Fig 3C

Fig 3D

Fig. 4

Fig 5 A

Fig B-C

Fig 5D

Fig 5E

Fig. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2015088039 A
- EP 3698133 A1
- WO 2014169336 A

### Non-patent literature cited in the description

- **BARGMANN et al.** Odorant-selective genes and neurons mediate olfaction in C. elegans. *Cell*, 13 August 1993, vol. 74, 515-527
- **IWANIR, S et al.** Irrational behavior in c. elegans arises from asymmetric modulatory effects within single sensory neurons. *Nat. communications*, 2019, vol. 10, 3202
- **COHEN, D. et al.** Bounded rationality in c. elegans is explained by circuit-specific normalization in chemosensory pathways. *Nat. communications*, 2019, vol. 10, 1-12
- **HIROTSU, T. et al.** A highly accurate inclusive cancer screening test using Caenorhabditis elegans scent detection. *PLoS One*, 2015, vol. 10, e0118699
- **UEDA, YUJI et al.** Application of C. elegans cancer screening test for the detection of pancreatic tumour in genetically engineered mice. *Oncotarget*, 2019, vol. 10 (52), 5412
- **DRAPER et al.** Menstrual cycle rhythmicity: metabolic patterns in healthy women. *SCIENTIFIC REPORTS*, 01 October 2018, vol. 8 (1)
- **DRAPER, C et al.** Menstrual cycle rhythmicity: metabolic patterns in healthy women. *Sci. reports*, 2018, vol. 8, 14568
- **DRAPER, C. et al.** Menstrual cycle rhythmicity: metabolic patterns in healthy women. *Sci. reports*, 2018, vol. 8, 14568
- **HIROTSU, T. et al.** *PLoS One*, 2015, vol. 10, e0118699
- **ALBRECHT ; BARGMANN**. High-content behavioral analysis of Caenorabditis elegans in precise spatiotemporal chemical environments. *Nature methods*, 2011
- **ADRIANA SAN-MIGUEL ; HANG LU**. Microfluidics as a tool for C. elegans research. *Wormbook.org*, 2013, 3
- Molecular Cloning: A Laboratory Manual. Cold Spring Harbor Laboratory Press, 2012
- **MELLO, C. C. ; KRAMER, J. M. ; STINCHCOMB, D. ; AMBROS, V.** Efficient gene transfer in C. elegans: extrachromosomal maintenance and integration of transforming sequences. *EMBO J*, 1991, vol. 10, 3959-3970
- **KAPOLKA et al.** DCyFIR: a high-throughput CRISPR platform for multiplexed G protein-coupled receptor profiling and ligand discovery. *PNAS*, 2020, vol. 117 (23), 13117-13126